# EUROPEAN PATENT APPLICATION

(11) **EP 1 421 896 A2**
(43) Date of publication of application: **26.05.2004**
(21) Application number: 03257236.4
(22) Date of filing: 17.11.2003
(51) Int. Cl.: A61B 5/00

(54) **Living body information detecting terminal control system**

(30) Priority: 19.11.2002 JP 2002335282; 27.12.2002 JP 2002379893; 03.10.2003 JP 2003346060
(71) Applicant: Seiko Instruments Inc., Chiba-shi, Chiba (JP)
(72) Inventor: Ozaki, Toru, Chiba-shi Chiba (JP); Ishii, Tomotada, Chiba-shi Chiba (JP); Iijima, Ryuji, Chiba-shi Chiba (JP); Moriya, Koichi, Chiba-shi Chiba (JP)
(74) Representative: Sturt, Clifford Mark

(57) **Abstract**

A living body information detecting terminal (2) is controlled from the side of a living body information monitoring device (3) connected to the living body information detecting terminal (2). In particular, a first living body information detecting sensor that is driven at all times or a second living body information detecting sensor that is in a power-save state or performs measurement in an intermittent manner during normal conditions, is selected, determined, and controlled from among living body information detecting sensors (5) provided to the living body information detecting terminal (2) depending on its wearer, and the second living body information detecting sensor is operated with transmission of an abnormality signal from the first living body information detecting sensor as a trigger. As a result, reductions in power consumption and data volume are attained, and a living body information detecting terminal control system with improved reliability is realized by verifying, at regular time intervals, the operation condition of the living body detecting sensor that is in a power-save state during normal conditions.

## Description

The present invention relates to a living body information detecting terminal control system which detects living body information of a user in his or her daily life regardless of normal/abnormal conditions and is useful for home health care and emergency notification.

As conventional systems designed for home health care and emergency notification, there exists one including a portable terminal that detects living body information and a living body information monitoring device that receives data transmitted from the portable terminal and performs processing on the data, in which the living body information and an emergency signal transmitted from the living body information monitoring device are received by an external host server via a public line to thereby accumulate data and respond to the emergency situation.

For example, the prior art disclosed in JP 2001-93068 A (pages 5 and 6, Fig. 1) describes a human body abnormality detecting communication terminal including: a moisture absorption detector A that detects falling of a wearer of the terminal (hereinafter simply referred to as the "wearer") into a river or a bathtub; an inclination detector C that detects tumbling of a human body; a human body wearing state detector B that detects a wearing state of the terminal by measuring a body temperature on a human body surface; and a human body abnormality judgement detector that operates a blood oxygen measuring device only when judging an abnormality from the respective outputs of the moisture absorption detector A and the human body wearing state detector B or from the respective outputs of the inclination detector C and the human body wearing state detector B, and when the obtained measurement output is abnormal, notifies this to a security service via a telephone line (see JP 2001-93068 A).

However, the system including the human body abnormality judgement detector and the human body abnormality detecting communication terminal that are described above (JP 2001-93068 A) is subject to the following problem. That is, the moisture absorption detector A, the human body wearing state detector B, and the inclination detector C which serve as triggers and transmit trigger signals, and control means for detecting the trigger signals to generate a control signal for operating the blood oxygen measuring device, are constructed as the same single terminal. Thus, in the case where the measurement output indicates no signal or a normal value even under an abnormal condition due to such factors as a breakdown of the blood oxygen measuring device or the control means, the abnormal condition is not communicated to the security service, rendering the system rather poor in terms of reliability.

Although the reliability of the system is improved if all the living body information detecting sensors connected to the living body information detecting device worn on the wearer are in the operating state at all times, this disadvantageously leads to increases in power consumption, data volume, etc.

The present invention has been made in view of the above-mentioned problems, and therefore has an object of the invention to provide a living body information detecting terminal control system including: a portable type living body information detecting terminal for temporarily storing a first data as a signal or transmitting the signal to the exterior as a first transmission data, the first data being constituted by a living body digital signal obtained by performing an A/D conversion on living body information detected by living body information detecting sensors or by a first processed data obtained by performing data processing on the living body digital signal; a living body information monitoring device for receiving the first transmission data transmitted from the living body detecting terminal and for temporarily storing a second data as a signal or transmitting the signal to the exterior as a second transmission data via a public line, the second data being constituted by a second processed data obtained by performing processing on the first data contained in the first transmission data or by the living body digital information; and a host server for receiving the second transmission data transmitted from the living body information monitoring device via the public line and for displaying or storing the second transmission data. The living body information detecting terminal control system is configured such that the living body information detecting terminal and the living body information monitoring device are capable of performing bidirectional communication with each other and that the living body information monitoring device controls the operation of the living body information detecting terminal by using, as a trigger, the signal received from the living body information detecting terminal. Accordingly, with the living body information detecting terminal control system according to the present invention, the drive conditions of the sensors provided to the living body information detecting terminal can be controlled from, for example, the living body information detecting device side.

The first transmission data is at least one of a first normal-time transmission data and a first abnormal-time transmission data. The first normal-time transmission data is constituted by at least one of the first data and identification information that is specific to the wearer and recorded in the living body information detecting terminal. The first abnormal-time transmission data is constituted by the first data, the identification information that is specific to the wearer and recorded in the living body information detecting terminal, and a signal indicating occurrence of an abnormality. Further, the second transmission data is at least one of a second normal-time transmission data and a second abnormal-time transmission data. The second normal-time transmission data is similarly constituted by at least one of the second data, the identification information that is specific to the wearer and recorded in the living body information monitoring device, and identification information for the device. The second abnormal-time transmission data is constituted by at least one of the second data, the identification information that is specific to the wearer and recorded in the living body information monitoring device, the identification information for the device, and the signal indicating occurrence of an abnormality.

Further, the living body information detecting terminal records a preset value range for the first data, and may be endowed with: a normal-time living body information transmitting function for temporarily storing the first data or transmitting the data to the living body information monitoring device at regular time intervals when the first data is within the preset value range; and an abnormal-time automatically notifying function for immediately performing communication with the outside when the first data is not within the preset value range.

Further, in a construction provided with a living body information detecting terminal control unit that generates a signal for controlling the living body information detecting terminal, the living body information monitoring device can receive the identification information that is specific to the wearer and stored in the living body information detecting terminal and send a control signal to the living body information detecting terminal by selecting, from among the living body information detecting sensors provided to the living body information detecting terminal, a first living body information detecting sensor that is driven under normal conditions or a second living body information detecting sensor that is in a power-save state or driven intermittently under normal conditions.

The selection between the first living body information detecting sensor and the second living body information detecting sensor and the sending of the control signal may be performed by the living body information detecting terminal.

Further, when an output from the first sensor, which is provided to the living body information detecting terminal and is driven during normal conditions, is not within a preset value range, the living body information monitoring device can transmit to the living body information detecting terminal a control signal for driving the second living body information detecting sensor that is in a power-save state during the normal conditions. Alternatively, in the case where the second living body information detecting sensor is driven intermittently during normal conditions, when the output from the first living body information detecting sensor is not within the preset value range, the living body information monitoring device may perform control on a measuring interval, a measuring time, and a data sampling frequency for the second living body information detecting sensor.

Further, the first living body information detecting sensor and the second living body information detecting sensor may be controlled by the living body information detecting terminal.

In addition, at least one call button is provided to the living body information detecting terminal or the living body information monitoring device. With pressing of the call button as a trigger, it is possible to perform the selection between the first living body information detecting sensor and the second living body information detecting sensor, and the control of the drive condition and the measurement operation of the second living body information detecting sensor.

Further, the living body information monitoring device can send, to the living body information detecting terminal, operation verification signals verifying the breakdown conditions or drive conditions of the respective sensors provided to the living body information monitoring device, and the living body information detecting terminal having received the operation verification signals can verify the breakdown conditions and the drive conditions and send them to the living body information monitoring device.

Further, it may be the host server that sends: the control signal for controlling the living body information detecting terminal by determining between the first living body information detecting sensor and the second living body information detecting sensor; the control signal for controlling the living body information detecting terminal by determining the drive condition and the measurement operation of the second living body information detecting sensor, which is in a power-save state or driven intermittently during normal conditions, based on an output from the first living body information detecting sensor that is driven under normal conditions; and the control signal for verifying the breakdown condition and the drive condition of the second living body information detecting sensor that is in a power-save state or driven intermittently during normal conditions.

Further, to solve the above-mentioned problem, according to a first aspect of the present invention, there is provided a living body information detecting terminal control system, including:
a living body information detecting terminal including:
   at least one living body information detecting sensor, the living body information detecting sensor serving to detect a living body information signal of a wearer; and
   means for transmitting living body information data to a living body information monitoring device;
the living body information monitoring device that, after receiving the living body information data transmitted from the living body information detecting terminal, stores the living body information data'in a storage device within the living body information monitoring device for a predetermined time, and includes means for transmitting the living body information data to a host server through a public line; and
the host server that, after receiving the living body information data transmitted from the living body information monitoring device, stores the living body information data in a storage device within the host server, or displaying the living body information data on a display device connected to the host server directly or through a network,
in which the living body information detecting terminal control system controls an operation of the living body information detecting terminal itself after the living body information detecting terminal detects the living body information signal.

According to a second aspect of the present invention, there is provided a living body information detecting terminal control system, including:
a living body information detecting terminal including:
   at least one living body information detecting sensor, the living body information detecting sensor serving to detect a living body information signal of a wearer; and
   means for transmitting living body information data to a living body information monitoring device;
the living body information monitoring device that, after receiving the living body information data transmitted from the living body information detecting terminal, stores the living body information data in a storage device within the living body information monitoring device for a predetermined time, and includes means for transmitting the living body information data to a host server through a public line; and
the host server that, after receiving the living body information data transmitted from the living body information monitoring device, stores the living body information data in a storage device within the host server, or displaying the living body information data on a display device connected to the host server directly or through a network,
in which, after receiving the living body information data from the living body information detecting terminal, the living body information monitoring device transmits living body information detecting terminal control data, which is used to control drive of the living body information detecting sensor of the living body information detecting terminal, to the living body information detecting terminal.

According to a third aspect of the present invention, in the second aspect of the invention, there is provided a living body information detecting terminal control system, in which the living body information detecting terminal further includes:
at least one living body information detecting sensor;
an A/D converter unit for converting a living body analog signal obtained from the living body information detecting sensor into a living body digital signal;
a living body digital signal control unit for processing the living body digital signal to prepare living body digital signal processed data, and performing control of the living body digital signal;
a ROM having a data structure including at least one of wearer identification information specific to the wearer, a living body information operational expression, and living body information detecting terminal identification information;
a first memory unit including a RAM for storing living body digital data composed of the living body digital signal or the living body digital signal processed data for a predetermined time;
a living body information sensor control unit for controlling drive of each living body information detecting sensor composing a living body information detecting sensor unit, based on the living body information detecting terminal control data received from the living body information monitoring device or a control content set with respect to the living body information detecting terminal by the wearer;
a living body information data transmitter/receiver unit for transmitting or receiving data to or from the living body information monitoring device; and
a first central processing unit for controlling the A/D converter unit, the first memory unit, the living body digital signal control unit, the living body information sensor control unit, and the living body information data transmitter/receiver unit.

According to a fourth aspect of the present invention, in the third aspect of the invention, there is provided a living body information detecting terminal control system, in which:
the living body information detecting terminal further includes:
   a first living body information data judging unit for comparing the living body digital data with a preset value range and outputting a comparison result; and
   a living body information detecting sensor control unit for controlling the drive of the living body information detecting sensor based on the comparison result; and
the living body information detecting sensor control unit controls each living body information detecting sensor based on living body information detecting terminal control data received from the living body information monitoring device, a judgment value obtained from the first living body information data judging unit, or the control content set by the wearer.

According to a fifth aspect of the present invention, in the fourth aspect of the invention, there is provided a living body information detecting terminal control system, in which the living body digital signal control unit of the living body information detecting terminal includes:
a living body digital data processing function for performing data processing on the living body digital signal; and
a normal-time living body information transmitting function for storing the living body digital signal processed data obtained by the living body digital data processing function or living body digital data composed of the living body digital signal in the RAM for a predetermined time, and at a predetermined timing or when receiving a polling command transmitted by the living body information monitoring device, transmitting a first normal-time transmission data composed of at least one of the living body digital data and the wearer identification information to the living body information monitoring device.

According to a sixth aspect of the present invention, in the fifth aspect of the invention, there is provided a living body information detecting terminal control system, in which the living body information detecting terminal further includes an abnormal-time living body information transmitting function for:
in a case where a value of the living body digital data is judged to be outside the preset value range in the first living body information data judging unit, outputting an abnormality signal indicating an abnormality from the first living body information data judging unit of the living body information detecting terminal, and immediately transmitting a first abnormal-time transmission data composed of the abnormality signal, the wearer identification information, and the living body digital data; and
in a case where the value of the living body digital data is judged to be within the preset value range in the first living body information data judging unit, deleting the abnormal-time transmission data, or storing the abnormal-time transmission data in the RAM for a predetermined time, and at a predetermined timing or when receiving the polling command transmitted by the living body information monitoring device, transmitting the first normal-time transmission data to the living body information monitoring device.

According to a seventh aspect of the present invention, in the second aspect of the invention, there is provided a living body information detecting terminal control system, in which the living body information detecting terminal further includes:
at least one normal-time driving living body information detecting sensor that is driven during normal conditions; and
at least one power-save living body information detecting sensor that is laid in a power-save state with no power supply during normal conditions.

According to an eighth aspect of the present invention, in the second aspect of the invention, there is provided a living body information detecting terminal control system, in which the living body information detecting terminal further includes:
at least one normal-time driving living body information detecting sensor that is driven during normal conditions; and
at least one power-save living body information detecting sensor that is intermittently driven during normal conditions.

According to a ninth aspect of the present invention, in the seventh aspect of the invention, there is provided a living body information detecting terminal control system, in which, in a case where an output value of the first living body information data judging unit is an abnormal output value, the living body information sensor control unit supplies power to the power-save living body information detecting sensor and drives the power-save living body information detecting sensor.

According to a tenth aspect of the present invention, in the eighth aspect of the invention, there is provided a living body information detecting terminal control system, in which, in a case where an output value of the first living body information data judging unit is an abnormal output value, the living body information sensor control unit performs control by a control method based on at least one of a measuring interval, a measuring time, and a data sampling frequency for an intermittent driving living body information detecting sensor.

According to an eleventh aspect of the present invention, in the second aspect of the invention, there is provided a living body information detecting terminal control, in which the living body information detecting terminal selects a first living body information detecting sensor from the living body information sensors based on an output result from the first living body information data judging unit, and transmits the selection result to the living body information monitoring device through the living body information data transmitter/receiver unit.

According to a twelfth aspect of the present invention, in the second aspect of the invention, there is provided a living body information detecting terminal control system, in which the living body information detecting terminal further includes a call button that enables a sensor operation control by being pressed.

According to a thirteenth aspect of the present invention, in the second aspect of the invention, there is provided a living body information detecting terminal control system, in which the living body information detecting terminal transmits the living body information data to the living body information monitoring device based on the living body information signal detected by the living body information detecting sensor, or controls the drive of the living body information detecting sensor based on an instruction from the living body information detecting terminal itself.

According to a fourteenth aspect of the present invention, in the second aspect of the invention, there is provided a living body information detecting terminal control system, in which at least one living body information detecting sensor of sensors composing a living body information detecting sensor unit of the living body information detecting terminal is a sensor for detecting a pulse.

According to a fifteenth aspect of the present invention, in the second aspect of the invention, there is provided a living body information detecting terminal control system, in which at least one living body information detecting sensor of sensors composing a living body information detecting sensor unit of the living body information detecting terminal is a sensor for detecting a blood sugar level or a blood glucose concentration.

According to a sixteenth aspect of the present invention, in the second aspect of the invention, there is provided a living body information detecting terminal control system, in which at least one living body information detecting sensor of sensors composing a living body information detecting sensor unit of the living body information detecting terminal is a sensor for detecting a moving state of the wearer.

According to a seventeenth aspect of the present invention, in the second aspect of the invention, there is provided a living body information detecting terminal control system, in which the living body information monitoring device includes:
a second transmitter/receiver unit for transmitting/receiving the living body information data to/from the living body information detecting terminal;
a living body digital signal processed data preparing unit for processing the living body digital data received from each living body information detecting sensor to prepare a second living body digital signal processed data;
a second memory unit including:
   a ROM having data with a data structure composed of at least one of:
      living body information monitoring device identification information specific to the living body information monitoring device;
      an operational expression for data processing;
      a value range preset for the living body information data received from each living body information detecting sensor or second living body information data composed of the second living body information processed data; and
      a table for identifying the wearer based on the wearer identification information that is recorded in the ROM of the living body information detecting terminal; and
   a RAM for storing the second living body information data for a predetermined time;
a second living body information data judging unit for judging a control method for the living body information monitoring device, and when receiving a first abnormal-time transmission data transmitted by the living body information monitoring device, comparing the second living body information data with the preset value range stored in the ROM for judgment;
a first living body information detecting terminal control unit that prepares living body information detecting terminal control data for controlling the living body information detecting terminal based on a judgment result from the second living body information data judging unit;
a first public line connecting unit for connecting to the host server through a public line; and
a second central processing unit for controlling the second transmitter/receiver unit, the second memory unit, the second judging unit, the second signal control unit, the first living body information detecting terminal control unit, and the first public line connecting unit.

According to an eighteenth aspect of the present invention, in the seventeenth aspect of the invention, there is provided a living body information detecting terminal control system, in which:
when the second living body information data is judged as being outside the value range preset by the second living body information data judging unit, the living body information monitoring device transmits a signal indicating an abnormality and a second abnormal-time transmission data composed of the wearer identification information, the living body information detecting terminal identification information, and the second living body information data, to the host server through the first public line connecting unit; and
when the second living body information data is judged as being within the value range preset by the second living body information data judging unit, the living body information monitoring device stores the second living body information data in the RAM for a predetermined time, and at a predetermined timing or in accordance with reception of a polling command received from the host server, transmits a second living body information data stored in the RAM and a second normal-time transmission data composed of the wearer identification information and the living body information detecting terminal identification information, to the host server through the first public line connecting unit.

According to a nineteenth aspect of the present invention, in the seventeenth aspect of the invention, there is provided a living body information detecting terminal control system, in which, in the living body information monitoring device in a case where the second living body information data is judged as being outside the value range preset by the second living body information data judging unit, and the second abnormal-time transmission data is transmitted to the host server:
the second living body information data judging unit has a function of determining an abnormal level taking as a criterion at least one of a type of the living body information detecting sensor exhibiting an abnormality and a differential of the second living body information data with respect to the preset value range;
the living body digital signal processed data preparing unit adds an abnormality signal corresponding to the abnormal level to the second abnormal-time transmission data; and
the second transmitter/receiver unit transmits the second abnormal-time transmission data.

According to a twentieth aspect of the present invention, in the seventeenth aspect of the invention, there is provided a living body information detecting terminal control system, in which:
the first living body information detecting terminal control unit includes a first sensor determining means for, based on the wearer identification information received from the second transmitter/receiver unit, selecting and determining one of a first normal-time driving living body information detecting sensor and the second living body information detecting sensor that is in a power-save state with no power supplied during normal conditions or is intermittently driven, from among the living body information detecting sensors included in the living body information detecting terminal; and
the first living body information detecting terminal control unit transmits the second abnormal-time transmission data to the living body information detecting terminal based on a determination result from the sensor determining means.

According to a twenty-first aspect of the present invention, in the seventeenth aspect of the invention, there is provided a living body information detecting terminal control system, in which the first living body information detecting terminal control unit further includes:
a first determining means for, when the second transmitter/receiver unit receives the first abnormal-time transmission data from the living body information detecting terminal, selecting and determining the living body information detecting sensor to be driven from among the second living body information detecting sensors that are included in the living body information detecting terminal and are in a power-save state during normal conditions; and
a first control signal generating means for generating a signal that drives the selected second living body information detecting sensor.

According to a twenty-second aspect of the present invention, in the seventeenth aspect of the invention, there is provided a living body information detecting terminal control system, in which the first living body information detecting terminal control unit further includes:
a first determining means for, when the second transmitter/receiver unit receives the first abnormal-time transmission data from the living body information detecting terminal, selecting and determining the living body information detecting sensor to be changed in at least one of a measuring interval, a measuring time, and a data sampling frequency, from among the second living body information detecting sensors that are intermittently driven during normal conditions within the living body information detecting terminal; and
a first control signal generating means for, based on a result from the first determining means, generating a signal for changing at least one of the measuring interval, the measuring time, and the data sampling frequency of the selected second living body information detecting sensor.

According to a twenty-third aspect of the present invention, in the seventeenth aspect of the invention, there is provided a living body information detecting terminal control system, in which the living body information monitoring device further includes a call button that enables operation control of a sensor by being pressed.

According to a twenty-fourth aspect of the present invention, in the second aspect of the invention, there is provided a living body information detecting terminal control system, in which the living body information monitoring device performs a sensor operation control in response to a signal detected by the living body information detecting terminal.

According to a twenty-fifth aspect of the present invention, in the second aspect of the invention, there is provided a living body information detecting terminal control system, in which, when an output from the first living body information detecting sensor is judged as being outside the preset value range, the living body information sensor control unit within the living body information detecting terminal or the first living body information detecting terminal control unit within the living body information monitoring device performs a control such that a measuring interval of the second living body information detecting sensor becomes shorter than the measuring interval required during normal conditions.

According to a twenty-sixth aspect of the present invention, in the second aspect of the invention, there is provided a living body information detecting terminal control system, in which, when an output from the first living body information detecting sensor is judged as being outside the preset value range, the living body information sensor control unit within the living body information detecting terminal or the first living body information detecting terminal control unit within the living body information monitoring device performs a control such that a measuring time of the second living body information detecting sensor becomes longer than the measuring time required during normal conditions.

According to a twenty-seventh aspect of the present invention, in the second aspect of the invention, there is provided a living body information detecting terminal control system, in which, when an output from the first living body information detecting sensor is judged as being abnormal, the living body information sensor control unit within the living body information detecting terminal or the first living body information detecting terminal control unit within the living body information monitoring device performs a control such that a data sampling frequency of the second living body information detecting sensor becomes higher than the data sampling frequency required during normal conditions.

According to a twenty-eighth aspect of the present invention, in the second aspect of the invention, there is provided a living body information detecting terminal control system, in which the living body information sensor control unit included in the living body information detecting terminal or the first living body information detecting terminal control unit included in the living body information monitoring device causes control of the second living body information detecting sensor to return to a preset reference control state, in a case where an output from the first living body information detecting sensor is judged as being outside the preset value range by the first judging unit or the second judging unit, and the output from the first living body information detecting sensor is again judged as being within the preset value range with respect to the second living body information detecting sensor in which at least one of the measuring interval, the measuring time, and the data sampling frequency is controlled.

According to a twenty-ninth aspect of the present invention, in the second aspect of the invention, there is provided a living body information detecting terminal control system, in which the first living body information detecting terminal control unit further includes:
a first sensor operation verification signal generating means for generating a verification signal that serves to regularly verify a breakdown condition or a drive condition of each living body information detecting sensor within the living body information detecting terminal; and
a first drive condition judging means for, after the living body information detecting sensor control unit included in the living body information detecting terminal verifies a breakdown condition or a drive condition of each living body information detecting sensor in response to the operation verification signal and receives a sensor operation verification result signal to be transmitted, judging the breakdown condition or the drive condition of the sensor from the living body information detecting sensor operation verification result signal.

According to a thirtieth aspect of the present invention, in the second aspect of the invention, there is provided a living body information detecting terminal control system, in which the host server includes:
a third memory unit including:
   a ROM in which the wearer identification information transmitted from the living body information monitoring device, a table for identifying the wearer from the living body information monitoring device identification information, and the polling timing are previously recorded; and
   a RAM for storing a second living body information data transmitted from the living body information monitoring device;
a second public line connecting unit for receiving the second normal-time transmission data or the second abnormal-time transmission data that are transmitted from the living body information monitoring device through the public line, and transmitting a polling command to the living body information monitoring device at the polling timing previously recorded in the ROM provided to the host server;
a display unit for displaying the second normal-time transmission data or the abnormal-time transmission data that are received from the living body information monitoring device;
a third signal control unit for performing a signal control by determining whether the second normal-time transmission data or the abnormal-time transmission data that are received from the living body information monitoring device are stored in the memory unit or displayed on the display unit;
a second living body information detecting terminal control unit for generating a signal for controlling the living body information detecting terminal; and
a third central processing unit for controlling the third memory unit, the second public line connecting unit, the display unit, the third signal control unit, and the second living body information detecting terminal control unit.

According to a thirty-first aspect of the present invention, in the thirtieth aspect of the invention, there is provided a living body information detecting terminal control system, in which the second living body information detecting terminal control unit includes:
the second determining means for, based on the identification information specific to the living body information monitoring device and the wearer identification information that are received by the second public line connecting unit, determining one of the first living body information detecting sensor that is driven during normal conditions and the second living body information detecting sensor that is in a power-save state with no power supply or is intermittently driven during normal conditions, from among the living body information detecting sensors included in the living body information detecting terminal; and
the second control signal generating means for generating a sensor control signal that is transmitted directly to the living body information detecting terminal or transmitted to the living body information monitoring device.

According to a thirty-second aspect of the present invention, in the thirty-first aspect of the invention, there is provided a living body information detecting terminal control system, in which the second living body information detecting terminal control unit further includes:
a second living body information detecting sensor operation verification signal generating means for generating a verification signal that serves to regularly verify a breakdown condition or a drive condition of each living body information detecting sensor included in the living body information detecting terminal; and
a second drive condition judging means for, after the living body information detecting sensor control unit included in the living body information detecting terminal verifies a breakdown condition or a drive condition of each living body information detecting sensor in response to the operation verification signal and receives a living body information detecting sensor operation verification result signal to be transmitted, judging the breakdown condition or the drive condition of the sensor from the living body information detecting sensor operation verification result signal.

According to a thirty-third aspect of the present invention, in the thirty-second aspect of the invention, there is provided a living body information detecting terminal control system, in which, in a case where an output signal from the first living body information detecting sensor exceeds a preset reference value range, or in a case where a difference with respect to an output signal previously outputted from the living body information detecting sensor exceeds a preset variation range, the first judging unit included in the living body information detecting terminal or the second judging unit included in the living body information monitoring device judges the output signal to be abnormal.

According to the living body information detecting terminal control system of the present invention, the ON/OFF of the living body information detecting sensors provided to the living body information detecting terminal is controlled from the living body information monitoring device side on the basis of the wearer identification information transmitted from the living body information detecting terminal. As a result, even when the wearer needs different types of living body information, the living body information detecting terminals provided for this purpose may all be constructed in the same manner, making it possible to simplify the manufacturing process for the living body information detecting terminal.

According to the living body information detecting terminal control system of the present invention, the living body information detecting sensors provided to the living body information detecting terminal consist of the first living body information detecting sensor that is driven during normal conditions and the second living body information detecting sensor that is in a power-save state or driven intermittently during normal conditions, and the ON/OFF or the operation of the second living body information detecting sensor is controlled from the living body information monitoring device side in accordance with the output of the first living body information detecting sensor which is transmitted from the living body information detecting terminal. As a result, it is not necessary to drive all the living body information detecting sensors provided to the living body information detecting terminal at all times, making it possible to achieve reductions in power consumption and data volume.

According to the living body information detecting terminal control system of the present invention, the signals for verifying the drive conditions and breakdown conditions of the living body information detecting sensors provided to the living body information detecting terminal are transmitted from the living body information monitoring device side at regular time intervals, thereby making it possible to grasp, in particular, the breakdown condition of the living body information detecting sensor that is in a power-save state or performs measurement in an intermittent manner during normal conditions. As a result, it is possible to avoid a situation where the living body information detecting sensor is in the breakdown condition when the wearer falls into an abnormal state and it is necessary to drive the living body information detecting sensor that is in a power-save state during normal conditions, thereby making it possible to improve the reliability of the system.

Embodiments of the invention will now be described by way of further example only and with reference to the accompanying drawings, in which:
Fig. 1 is a block diagram showing the overall construction of a living body information detecting terminal control system of the present invention;
Fig. 2 is a block diagram showing a construction of a living body information detecting terminal in the living body information detecting terminal control system of the present invention;
Fig. 3 is a block diagram showing a modified example of the construction of the living body information detecting terminal in the living body information detecting terminal control system of the present invention;
Fig. 4 is a block diagram showing a construction of the living body information monitoring device in the living body information detecting terminal control system of the present invention;
Fig. 5 is a block diagram showing a modified example of the construction of the living body information monitoring device in the living body information detecting terminal control system of the present invention;
Fig. 6 is a block diagram showing a construction of a host server in the living body information detecting terminal control system of the present invention;
Fig. 7 is a block diagram showing a modified example of the construction of the host server in the living body information detecting terminal control system of the present invention;
Fig. 8 is a block diagram showing a modified example of the overall construction of the living body information detecting terminal control system of the present invention;
Fig. 9 is a block diagram showing a modified example of the overall construction of the living body information detecting terminal control system of the present invention;
Figs. 10A and 10B are diagrams for explaining a measuring interval, a measuring time, and a data sampling frequency for the living body information detecting terminal control system of the present invention;
Fig. 11 is a chart for explaining a control flow between the living body information detecting terminal and the living body information monitoring device according to the living body information detecting terminal control system of the present invention;
Fig. 12 is a chart for explaining a modified example of a control flow between the living body information detecting terminal and the living body information monitoring device according to the living body information detecting terminal control system of the present invention;
Figs. 13A to 13C are explanatory diagrams showing contents of data recorded in a memory unit provided to the living body information detecting terminal and the structure of the data according to the living body information detecting terminal control system of the present invention;
Figs. 14A to 14C are explanatory diagrams showing contents of data recorded in a memory unit provided to the living body information monitoring device and the structure of the data according to the living body information detecting terminal control system of the present invention;
Fig. 15 is a chart for explaining a judgment flow performed by the living body information detecting terminal according to the living body information detecting terminal control system of the present invention;
Fig. 16 is a chart for explaining a judgment flow performed by the living body information monitoring device according to the living body information detecting terminal control system of the present invention; and
Figs. 17A and 17B are explanatory diagrams showing contents of transmission data transmitted from the living body information detecting terminal to the living body information monitoring device and the structure of the transmission data according to the living body information detecting terminal control system of the present invention.

Preferable embodiments of the present invention will hereinafter be described in detail with reference to the accompanying drawings.

### First Embodiment

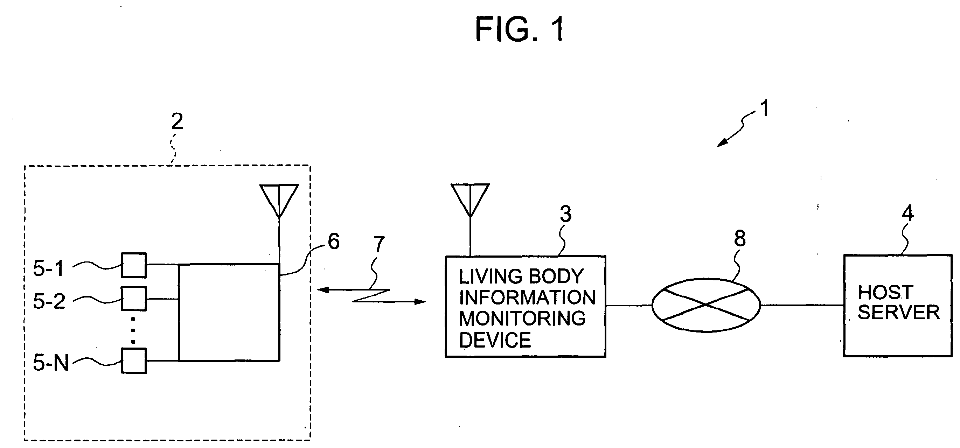

Fig. 1 is a block diagram showing a configuration of a living body information detecting terminal control system 1 of the present invention. This configuration is an example of a basic form of the present invention. In Fig. 1, roughly speaking, the living body information detecting terminal control system 1 of the present invention includes: a living body information detecting terminal 2 which will be described later and has at least one living body information detecting sensors 5-1 to 5-N and processing/transmitting means 6 and which will be described later; a living body information monitoring device 3 which will be described later and receives a first transmission data as a first normal-time transmission data or a first abnormal-time transmission data which is transmitted from the living body information detecting terminal 2; a host server 4 for receiving a second transmission data as a second normal-time transmission data or a second abnormal-time transmission data which is transmitted from the living body information monitoring device 3; bidirectional communication means 7 for establishing communication between the living body information detecting terminal 2 and the living body information monitoring device 3; and a public line 8. Here, the living body information detecting terminal 2 may adopt either a form of being carried by a user, or a form of a stationary type. Likewise, the living body information monitoring device 3 may adopt either a form of a portable type or a form of a stationary type. In addition, the bidirectional communication means 7 may be either of a wireless type or of a wired type irrespective of the communication system as long as the communication is bidirectionally carried out through the bidirectional communication means 7. Preferably, a wireless communication means such as an RFM system or Bluetooth™ can be used. For the public line 8, any public line can be used without a problem, such as a wired telephone line, a mobile phone line, a PHS line, or an optical LAN cable.

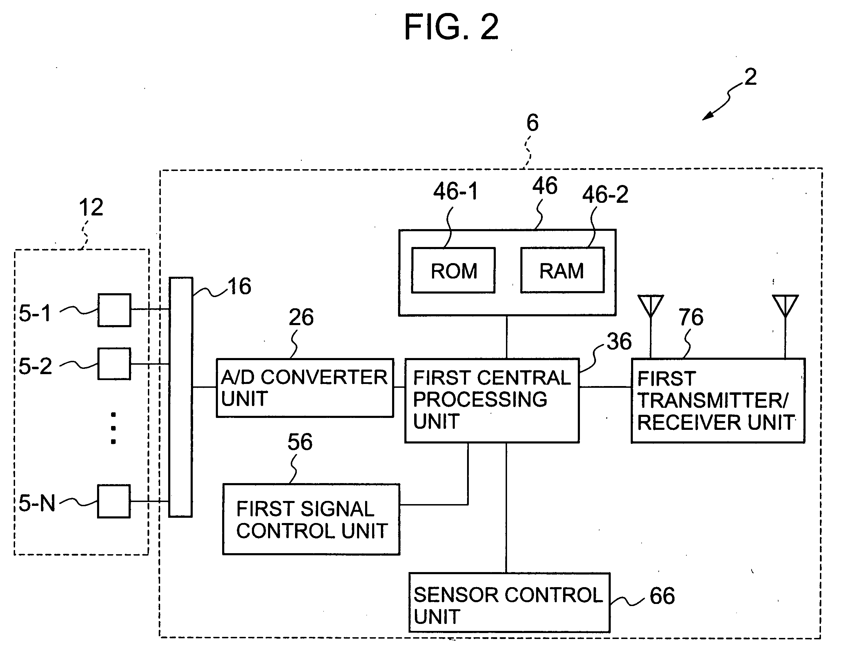

A first embodiment of configurations of the living body information detecting terminal 2, the living body information monitoring device 3, and the host server 4 will hereinbelow be described. Fig. 2 is a block diagram showing the configuration of the living body information detecting terminal 2. This configuration is an example of a basic form of the living body information detecting terminal in the living body information detecting terminal control system of the present invention.

In Fig. 2, the living body information detecting terminal 2 as a constituent element of the living body information detecting terminal control system 1 of the present invention includes: a sensor unit 12 having N sensor groups 5-1 to 5-N (where N is at least 1) for detecting living body information; a connector unit 16 for connecting the sensor unit 12 to a processing/transmitting/receiving unit 6; an A/D converter unit 26 for converting living body information obtained from the sensor unit 12 into a living body digital signal; a first signal control unit 56 for carrying out signal control by performing processing on the living body digital signal to generate a first processed data; a first memory unit 46 including a ROM 46-1 for storing therein data of a previously set value range for the first data consisting of the living body digital signal or the first processed data, and identification information specific to a wearer, and a RAM 46-2 for temporarily storing therein the first data; a sensor control unit 66 for controlling the living body information detecting sensors of the sensor unit 12 in accordance with a request or a command issued from the outside; a first transmitting/receiving unit 76 for transmitting/receiving a signal to/from the outside; and a first control processing unit 36 for controlling the A/D converter unit 26, the first memory unit 46, the first signal control unit 56, the sensor control unit 66, and the first transmitting/receiving unit 76.

Here, for the sensor unit 12, any sensors may be applied as long as they are sensors for detecting physiological information or movement information of the wearer such as a sphygmomanometer, a blood oxygen saturation measuring instrument, a skin impedance measuring instrument, a sweating amount measuring instrument, a weighing machine, a momentum measuring instrument, a clinical thermometer, and a glucose sensor for measuring a blood glucose concentration. For example, a sensor for detecting pulses or acceleration following movement may be used. Here, as for a sensor for detecting the pulses, any sensors may be applied as long as they are sensors for detecting pulses or heartbeats to convert the pulses or the heartbeats into an electrical signal, such as a sensor for driving a piezoelectric device as a transducer to transmit an ultrasonic wave to a living body to obtain a reflected wave to detect pulses from the reflected wave, a sensor for converting a heartbeat sound, which is obtained from a detector for detecting heartbeats such as a stethoscope used by a doctor, into an electrical signal to detect heartbeats or pulses, a sensor for detecting pulses at the tip of a finger or an earlobe on the basis of a photoelectric pulse wave system, and a sensor for detecting heartbeats from electrodes worn on the breast. In addition, as for the sensor for detecting acceleration, any sensors, including a piezoelectric type or an electrostatic type sensor, which are adapted to be able to detect acceleration may be applied. Moreover, for detection of the movement, an angular velocity or an angular acceleration may be detected in addition to acceleration.

In addition, an identification number specific to the wearer and/or a terminal may be recorded in the ROM 46-1 built in the first memory unit 46. In addition to this identification number, personal information such as an address, a name, age, a telephone number, a blood group, and anamnesis of a wearer, and a name and a contact address of a family doctor, data on the timing with which the first data detected by the sensor unit 12 and temporarily stored in the RAM 46-2 is transmitted from the first transmitting/receiving unit 76 to the living body information monitoring device or the server provided in the outside, and identification information used to specify the living body information monitoring device or the server as the destination to which the first data is sent may also be recorded in the ROM 46-1 built in the first memory unit 46.

The first signal control unit 56 is provided with a first data processing function of performing data processing on the living body digital signal, and a normal-time living body information transmitting function of transmitting a first normal-time transmission data, which will be described later, to the outside. First of all, the first data processing function will hereinbelow be described by giving an actual example. For the living body digital signal and the first processed data contained in the first data, when, for example, the detected living body information is a pulse, a pulse wave waveform detected by the sensor corresponds to the living body digital signal, and the number of pulses per minute which is obtained by calculating peak intervals of the pulse wave waveform corresponds to the first processed data. When the detected living body information is acceleration, an acceleration waveform detected by a sensor corresponds to the living body digital signal, and the number of pitches per minute which is obtained by calculating the peak intervals of the acceleration waveform, action information such as falling down of the wearer which is identified on the basis of a waveform, momentum which is calculated on the basis of a waveform, and the like correspond to the first processed data. With respect to any kind of sensor used for indicating a health state of the wearer typified by a blood pressure, blood oxygen saturation, a skin impedance, sweating, a weight, a body temperature, a blood glucose concentration, and the like, data obtained by performing digital conversion on raw data obtained from the living body information detecting sensor is the living body digital signal, and data obtained as a result of subjecting the living body digital signal to a processing that will be described later is the first processed data. Data containing at least any one of the living body digital signal and the first processed data is referred to as the first data. For a method of processing the living body digital signal, a statistical processing for obtaining a mean value, dispersion of data, a maximum value, a minimum value, and the like may be executed at arbitrary time intervals. In addition, a mathematical processing such as a fast Fourier transform, or a processing such as a waveform analysis may also be executed. The same as described above applies also to a living body digital signal and second processed data constituting a second data that will be described later.

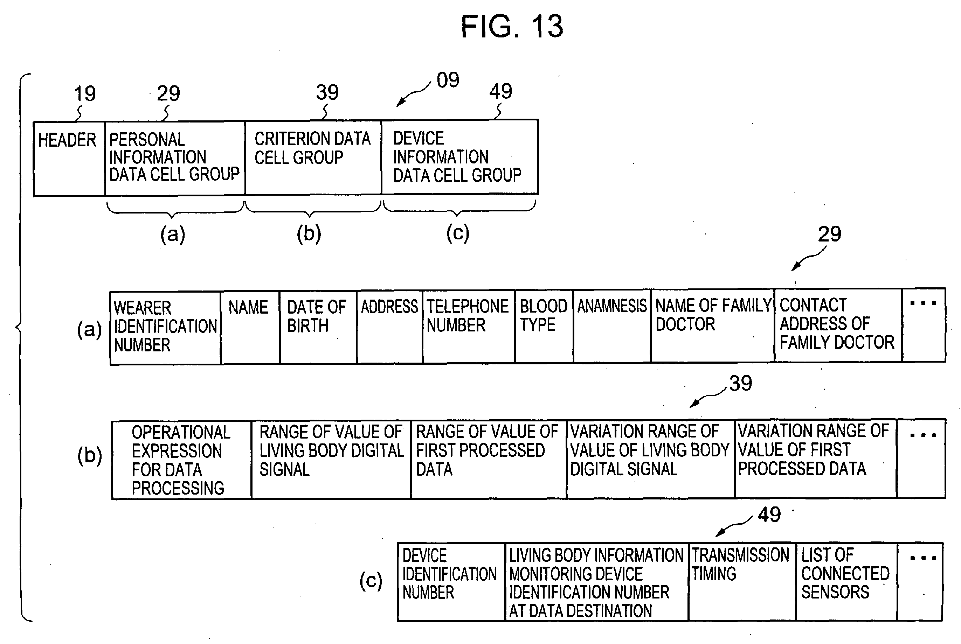

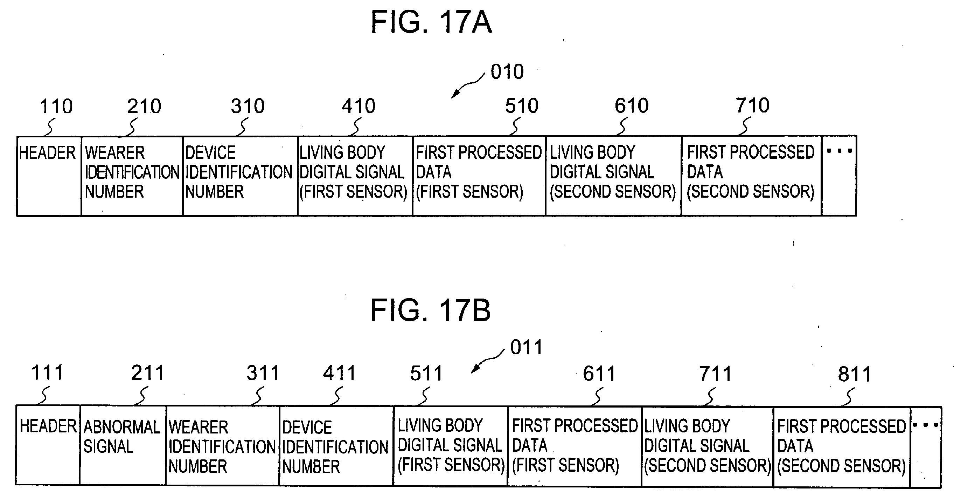

In addition, the first signal control unit 56 has a normal-time living body information transmitting function of carrying out signal control so as to periodically transmit a first normal-time transmission data 010 containing therein at least any one of the first data temporarily stored in RAM 46-2 and the predetermined identification information specific to the wearer which is recorded in ROM 46-1 as the first transmission data, from the first transmitting/receiving unit 76 to the living body information monitoring device or the server provided in the outside in accordance with a predetermined transmission timing which is recorded in the ROM 46-1. In addition to 'the transmission timing recorded in the ROM 46-1, the first normal-time transmission data may also be transmitted when a transmission request has been made by the living body information monitoring device or the server provided in the outside. One example of a data structure of the first normal-time transmission data 010 is shown in Fig. 17A. The first normal-time transmission may adopt a data structure in which the living body digital signal and the first processed data are continuously recorded for each sensor, for example, a structure in which a living body digital signal 410 from a first sensor, a first processed data 510 from the first sensor, a living body digital signal 610 from a second sensor, and a first processed data 710 from the second sensor are arranged after a header 110, a wearer identification number 210, and a device identification number 310. The data structure shown in Fig. 17A is merely one example. Thus, the order of the data shown in Fig. 17A may be changed. Also, as for the data structure, any data structure such as a hierarchical type, a network type, or a relational type structure may be adopted, and hence the data structure is not intended to be limited to the form shown in Figs. 13A to 13C.

In addition, the sensor control unit 66 carries out a control for the sensor groups 5-1 to 5-N included in the sensor unit 12, such that power is constantly supplied to the sensor determined as the first living body information detecting sensor to be driven, and no power is supplied to the sensor determined as the second living body information detecting sensor that is in a power-save state, in accordance with a request made by the external living body information monitoring device or server.

In addition, a determination may also be made such that the sensor control unit 66 performs control for the sensor groups 5-1 to 5-N included in the sensor unit 12 to drive the first living body information detecting sensor is driven at all times, and to intermittently drive the second living body information detecting sensor, in accordance with a request made by the external living body information monitoring device or server. In this case, the control may also be carried out such that the power is constantly supplied to the first living body information detecting sensor, and the measurement is intermittently carried out according to preset measuring intervals, measuring time and a data sampling frequency using the second living body information detecting sensor.

Moreover, a determination may also be made such that the sensor control unit 66 carries out a control for the sensor groups 5-1 to 5-N included in the sensor unit 12 to drive both of the first living body information detecting sensor and the second living body information detecting sensor intermittently in accordance with a request made by the external living body information monitoring device or server. In this case, the measuring interval of the first living body information detecting sensor is set so as to be equal to or shorter than that of the second living body information detecting sensor, the measuring time of the first living body information detecting sensor is set so as to be equal to or longer than that of the second living body information detecting sensor, and the data sampling frequency of the first living body information detecting sensor is set so as to be equal to or higher than that of the second living body information detecting sensor.

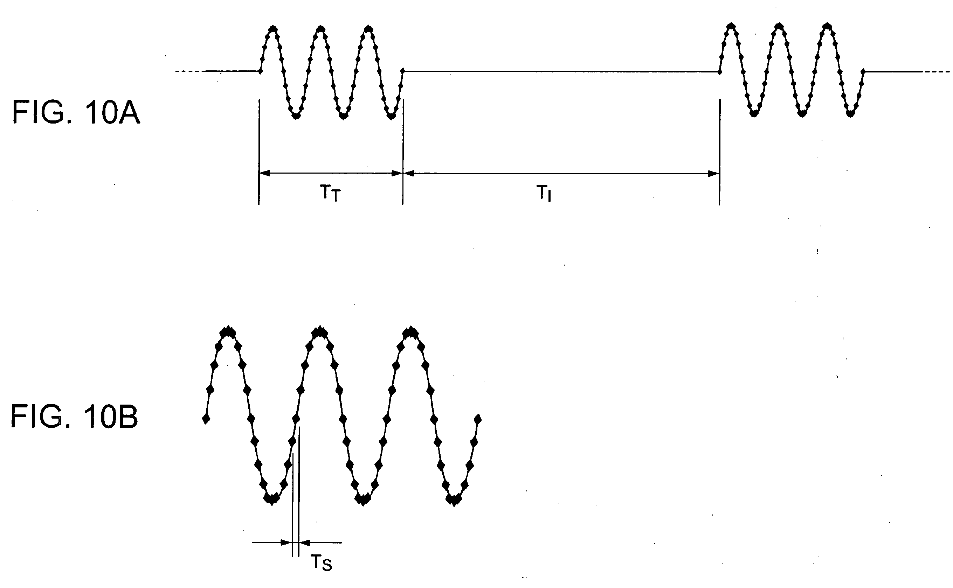

Figs. 10A and 10B show waveform charts useful in explaining the measuring intervals, the measuring time, and the data sampling frequency. Fig. 10A is one example showing a situation in which data is intermittently measured for a certain fixed time period, and Fig. 10B is an enlarged waveform chart of an area in which the data is measured in Fig. 10A. Here, the measuring interval refers to a time interval from the last measurement to the next measurement, and indicates a time period T_{I} in Fig. 10A. In addition, the measuring time refers to a time period required for one measurement, and indicates a time period T_{T} in Fig. 10A. Also, the data sampling frequency refers to a frequency of data acquisition during one measurement, and is expressed by a reciprocal number of a time period T_{S} in Fig. 10B.

As for a combination of the first living body information detecting sensor and the second living body information detecting sensor, for example, an action sensor including an acceleration sensor, an angular velocity sensor, and an angular acceleration sensor and serving to detect an action and a movement of the wearer may be used as the first living body information detecting sensor. Also, a sensor for detecting a circulatory movement such as a pulse sensor for detecting pulse information of the wearer, or a sphygmomanometer for detecting blood pressure information of the wearer may be used as the second living body information detecting sensor. Alternatively, while using the action sensor as the first living body information detecting sensor as described above, a blood glucose concentration sensor for detecting a blood glucose concentration, a blood-sugar level detecting sensor, or a clinical thermometer for detecting a body temperature may also be used as the second living body information detecting sensor. Moreover, a sensor for detecting a circulatory movement such as the pulse sensor or the blood pressure sensor may also be used as the first living body information detecting sensor, and the blood-sugar level detecting sensor or the clinical thermometer may also be used as the second living body information detecting sensor.

As described above, as long as a sensor for detecting a detection object having a large change rate of data over time is selected for the first living body information detecting sensor, and a living body information detecting sensor for detecting a detection object having a small change rate of data over time as compared with the detection object of the first living body information detecting sensor is selected for the second living body information detecting sensor, the present invention is not limited to the combination of the sensors given in the above-mentioned example. For example, as a modified example of the above-mentioned combination example, since an amount of movement of a body is extremely small while the wearer is asleep as compared with that in the daytime, and hence a change rate of data over time is small, a pulse sensor for detecting a detection object having a larger change rate may be selected as the first living body information detecting sensor, while an action sensor may be selected as the second living body information detecting sensor.

Moreover, in an example in which, during normal conditions, the first living body information detecting sensor and the second living body information detecting sensor are intermittently driven, as for a method of controlling operations of the living body information detecting sensors, such as the measuring intervals, the measuring time, and the data sampling frequencies of the first living body information detecting sensor and the second living body information detecting sensor, a predetermined operation control method may be used regardless of what type of sensor is selected as the living body information detecting sensor, or different operation control methods may be set for different types of the selected living body information detecting sensor.

Moreover, the selection between the first living body information detecting sensor and the second living body information detecting sensor, and the setting of the method of controlling operations of these living body information detecting sensors are carried out in response to a request made by the external living body information monitoring device or server. In addition to the above, a manipulation unit (not shown) may be provided in the living body information detecting terminal, and such selection and setting may also be directly made from the living body information detecting terminal.

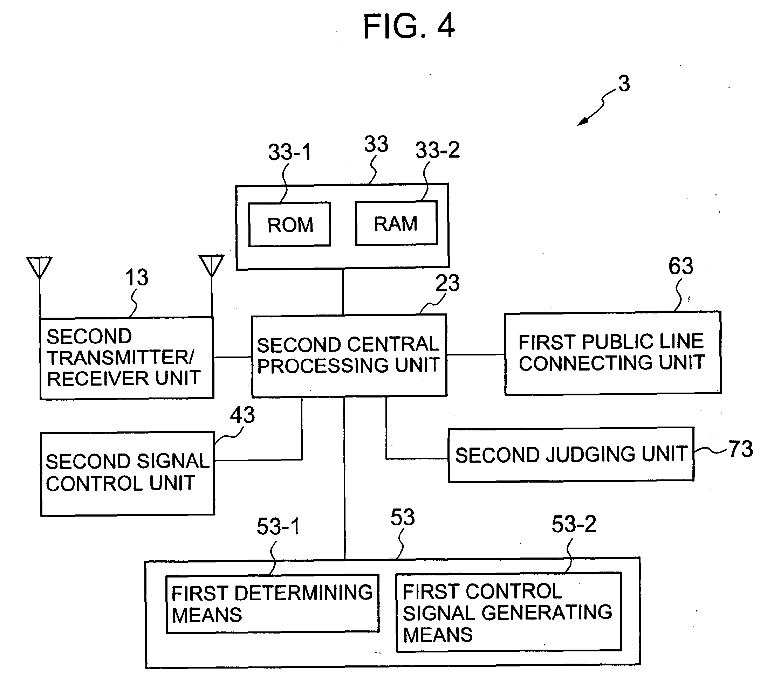

Fig. 4 is a block diagram showing a configuration of the above-mentioned living body information monitoring device 3 in the living body information detecting terminal control system of Fig. 1. This configuration is an example of a basic form of the living body information monitoring device in the living body information detecting terminal control system of the present invention.

In Fig. 4, the living body information monitoring device 3 as a constituent element of the living body information detecting terminal control system of the present invention includes: a second transmitting/receiving unit 13 for transmitting/receiving a signal to/from the above-mentioned living body information detecting terminal 2; a second judging unit 73 and a second central processing unit 23 for judging and processing the first normal-time transmission data received by the second transmitting/receiving unit 13; a second signal control unit 43 for carrying out signal control such that the received first data is processed to generate the second processed data; a second memory unit 33 including a ROM 33-1 in which a table provided for specifying the wearer and personal information of the wearer on the basis of the identification information specific to the wearer and received from the living body information detecting terminal 2, identification information specific to the living body information monitoring device, and data on a normal range of the second data containing therein the living body digital signal or the second processed data are to be recorded, and a RAM 33-2 for temporarily storing therein at least any one of the second data or the processing results obtained through the processing in the judging unit 73 and the second control processing unit 23; a first living body information detecting terminal control unit 53 including first detecting means 53-1 for selecting and determining any one of the first living body information detecting sensor and the second living body information detecting sensor with respect to each wearer of the living body information detecting terminal on the basis of the identification information which is specific to the wearer and received from the living body information detecting terminal, and first control signal generating means 53-2 for generating a signal used to transmit the obtained determination results to the living body information detecting terminal; and a first public line connecting unit 63 for transmitting/receiving a signal to/from the external server through a public line.

As for a method of processing the first data, a statistical processing may be executed with respect to a mean value, dispersion of data, a maximum value, a minimum value and the like at arbitrary time intervals, or a mathematical processing such as a fast Fourier transform, or a processing such as a waveform analysis may also be executed.

In addition, data on the timing of polling for the living body information detecting terminal may be recorded in the ROM 33-1 built in the secondary memory unit 33 in addition to the table and the identification number.

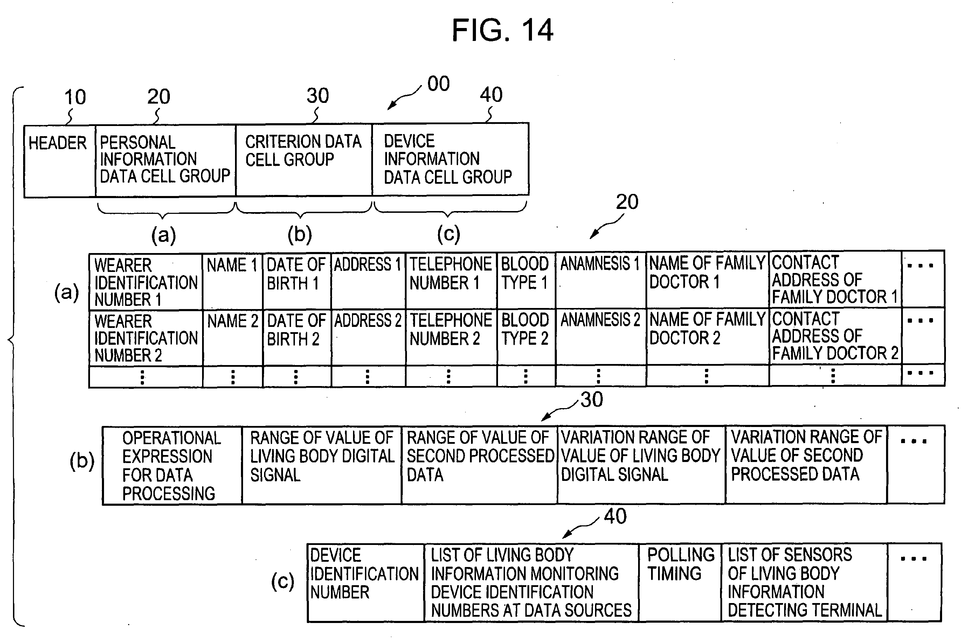

Figs. 14A to 14C show data 00 previously stored in the ROM 33-1 of the living body information monitoring device 2 and one example of its data structure. Roughly speaking, the data 00 contains therein at least one of a header 10, a personal information data cell group 20, a judgment criteria data cell group 30, and a device information data cell group 40. Of those, one example of the contents of the personal information data cell group 20 is shown in Fig. 14A. Personal information of the wearer such as a wearer identification number, a name, date of birth, an address, a telephone number, a blood group, and an anamnesis of the wearer, a name, a contact address and the like of a family doctor is, for example, recorded for every previously registered wearer in the personal information data cell group 20 to form a table used to specify the wearer and personal information of the wearer on the basis of the identification information which is specific to the wearer and received from the living body information detecting terminal 2. Also, likewise, one example of the contents of the judgment criteria data cell group 30 is shown in Fig. 14B. References serving as judgment criteria, such as an operational expression for data processing that will be described later, a value range of the living body digital signal, a value range of the second processed data, a variation range of a value of the living body digital signal, and a variation range of a value of the second processed data are, for example, recorded in the judgment criteria data cell group 30. Moreover, one example of the contents of the device information data cell group 40 is shown in Fig. 14C. An identification number of a device, a list of identification numbers of the living body information detecting terminals as data sources, data on the timing of polling for requesting the living body information detecting terminal 2 to transmit the first data temporarily stored in the RAM 46-2 of the living body information detecting terminal 2 to the living body information monitoring device, a list of the sensors connected to the living body information detecting terminal 2, and the like are, for example, recorded in the device information data cell group 40. The data contents and the data structure of Figs. 14A to 14C are merely one example. Thus, the data contents enumerated in Figs. 14A to 14C may be changed, or any data structure such as a hierarchical type, a network type, or a relational type structure may be adopted. Thus, the data structure and the like are not intended to be limited to the form shown in Figs. 14A to 14C.

In the second judging unit 73 and the second central processing unit 23, the judgment may or may not be carried out for the second data. When the judgment is carried out, data on a value range preset as a normal range of the second data is previously stored in the ROM 33-1, and this is compared with the second data to enable an abnormality judgment. A general normal range may be set as the normal range of the second data. For example, when the living body information to be detected is pulses, the normal range of the pulsation is from 50 pulses to 100 pulses per minute, when the living body information to be detected is a blood pressure, the normal range of a maximum blood pressure is equal to or lower than 130 mmHg, and the normal range of a minimum pressure is equal to or lower than 85 mmHg, and when the living body information to be detected is acceleration, the normal range of the acceleration is equal to or smaller than 2 G. Alternatively, using a standard deviation σ for a mean value M obtained from personal history of the second data of a wearer himself/herself which is recorded in a host server that will be described later, M + σ or M + 2σ, for example, may be set as the normal range, and then may be transmitted from the host server to the living body information monitoring device to be recorded in the memory unit 33. Moreover, an algorithm may also be used with which when a difference between a current output signal and the last output signal exceeds a preset variation range, the current output signal is judged to be abnormal.

In accordance with a determination criterion with which the first living body information detecting sensor or the second living body information detecting sensor is selected and determined by the first determination means, a server which is a passive sensor having less power consumption and which can detect an abnormal state of the wearer or a precursory condition of an abnormality is selected as the first living body information detecting sensor. Moreover, a sensor serving to detect data having a large change rate over time as compared with a detection object of the second living body information detecting sensor may be selected in some cases. For example, an acceleration sensor which can detect falling down of the wearer can be used as the first living body information detecting sensor. In addition, for example, for a diabetic, a blood-sugar level is the living body information required to be monitored for a long term, for a hypertension patient, a blood pressure is the living body information required to be monitored for a long term, and for a patient in rehabilitation for the limbs, acceleration is such living body information. Thus, considering that the living body information required to be monitored for a long term differs depending on the wearer, a sensor that is most required to carry out monitoring and measurement for that specific wearer may be set as the first living body information detecting sensor on the basis of the identification information specific to the wearer, and any other sensor may be selected and determined as the second living body information detecting sensor.

In addition, in the second signal control unit 43, signal control is carried out such that the second transmission data containing therein the second data temporarily stored in the RAM 33-2, the identification information that is specific to the wearer and recorded in the ROM 33-1, the identification information specific to the living body information monitoring device, and a signal indicating normality/abnormality judged in the judging unit 73 and the central processing unit 23 is periodically transmitted from the first public line connecting unit 63 to the external server in accordance with the transmission timing data recorded in the ROM 33-1. In addition to the timing data recorded in the ROM 33-1, the second transmission data may be transmitted when a transmission request has been made by the external server.

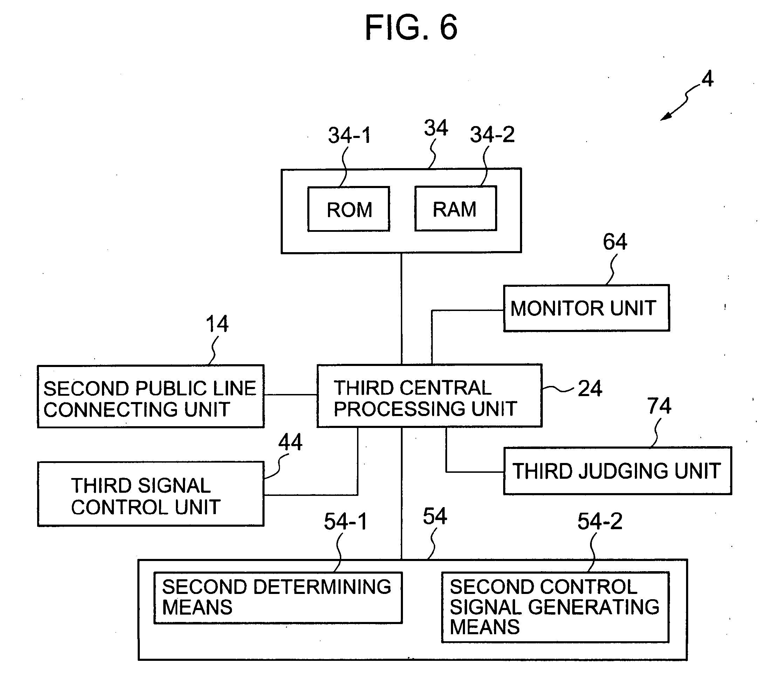

Fig. 6 is a block diagram showing a configuration of the host server 4 in the living body information detecting terminal control system of Fig. 1. This configuration is an example of a basic form of the host server in the living body information detecting terminal control system of the present invention.

In Fig. 6, the host server 4 as a constituent element of the living body information detecting terminal control system of the present invention includes: a second public line connecting unit 14 for transmitting/receiving a signal to/from the living body information monitoring device 3 through a public line; a third judging unit 74 and a third central processing unit 24 for judging and processing the second data contained in the second transmission data received from the second public line connecting unit 14; a third memory unit 34 including a ROM 34-1 in which a table provided for specifying the wearer and personal information of the wearer on the basis of the identification information that is specific to the wearer and received from the living body information monitoring device and/or the identification information specific to the living body information monitoring device, and the identification information specific to the host server are to be recorded, and a RAM 34-2 for storing therein the second data contained in the received second transmission data in order to form a database; a monitor unit 64 for displaying thereon at least any one of the second data or the judgment results obtained with the third judging unit 74 and the third processing unit 24 to visualize such data; a third signal control unit 44 for carrying out signal control by determining whether the second data and the judgment results are to be stored in a memory, displayed on a monitor, or both stored in the memory and displayed on the monitor; and a second living body information detecting terminal control unit 54 including second determining means 54-1 for selecting and determining any one of the first living body information detecting sensor and the second living body information detecting sensor for each wearer of the living body information detecting terminal on the basis of the identification information that is specific to the wearer and received from the living body information detecting terminal 2, and second control signal generating means 54-2 for generating a signal used to transmit the determination results to the living body information detecting terminal.

The monitor unit 64 may be directly connected to the above-mentioned living body information monitoring device, or may be a terminal unit connected through a network.

In addition, in the third judging unit 74 and the third central processing unit 24, data on a value range previously set as a normal range of the second data contained in the received second transmission data is previously recorded in the ROM 34-1, and this is compared with the second data to allow a judgment as to whether or not the range concerned is abnormal. Since the normal range of the second data is the same as that recorded in the ROM 33-1 of the above-mentioned living body information monitoring device 3, its description is omitted here for avoiding repetition.

A statistical processing for a mean value, dispersion of data, a maximum value, a minimum value and the like may be executed at arbitrary time intervals for the second data, or a mathematical processing such as a fast Fourier transform, or a processing such as a waveform analysis may also be executed for the second data.

### Second Embodiment

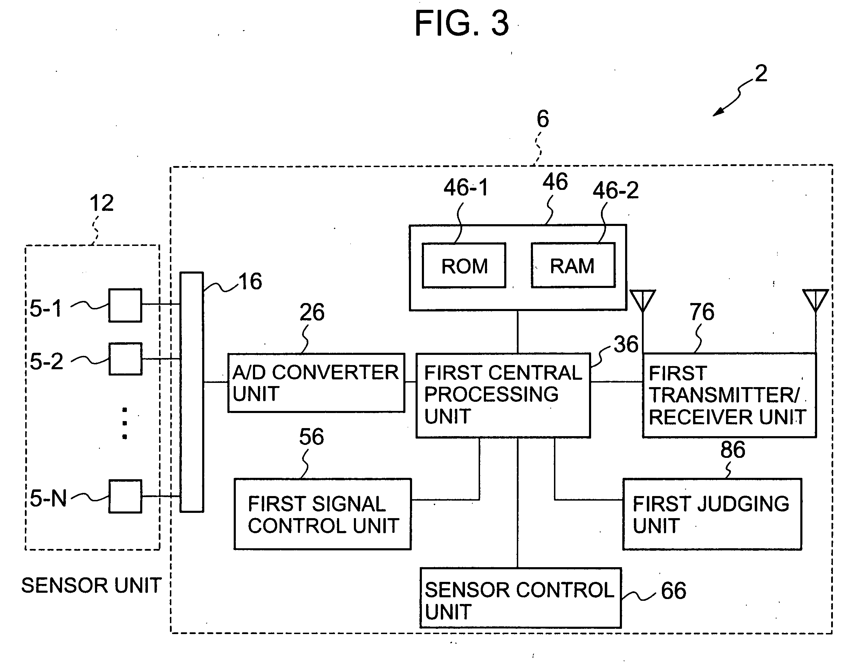

Since the overall configuration of a second embodiment of the invention is the same as that of the first embodiment shown in Fig. 1, its description is omitted here. The second embodiment of configurations of the living body information detecting terminal 2, the living body information monitoring device 3, and the host server 4 will hereinbelow be described. Fig. 3 is a block diagram showing a configuration of the living body information detecting terminal 2, and illustrates a modified example of the living body information detecting terminal in the living body information detecting terminal control system of the present invention.

As can be seen by comparing Fig. 3 with Fig. 2, the configuration shown in Fig. 3 is completely the same as that shown in Fig. 2 except that a first judging unit is provided in the living body information detecting terminal 2 of the second embodiment. Thus, constituent elements having the same configurations, functions and operations as those of the constituent elements shown in Fig. 2 are designated by the same reference numerals, and hence a description of the functions and the operations is omitted here in order to avoid a repetitive description. Here, a description will be given with respect to a first judging unit 86 having a configuration different from that of the living body information detecting terminal 2 shown in Fig. 2, and a sensor control unit 66, a first memory unit 46, and a first signal control unit 56 that operate differently from those of the living body information detecting terminal 2 shown in Fig. 2. The first judging unit 86 compares a first data, which is obtained from an output signal of the first living body information detecting sensor that is controlled so as to be driven at all times or intermittently driven by the sensor control unit 66, with data on a value range preset as a normal range and recorded in a ROM 46-1 which will be described later. Then, a signal transmission mode is changed on the basis of the judgment results in the first signal control unit 56 that will be described later. In addition, since the judgment criteria can be made identical to that used in the second judging unit 73 in the first embodiment, its description is omitted here in order to avoid a repetitive description.

If it is judged in the first judging unit 86 that the first data is outside the range of the preset value, then the first signal control unit 56 immediately carries out signal control so as to transmit a first abnormal-time transmission data containing therein at least any one of a signal indicating abnormality, the identification information specific to a wearer, and the first data to the outside. On the other hand, if it is judged in the first judging unit 86 that the first data falls within the range of the preset value, then similarly to the case of the first embodiment, the first signal control unit 56 carries out the signal control so as to transmit a first normal-time transmission data containing therein at least any one of a signal indicating normality, the identification information specific to a wearer, and the first data to the outside at a polling timing recorded in the ROM 46-1, or in accordance with a transmission request made by the living body information monitoring device 3.

Figs. 17A and 17B are diagrams showing a data structure of the first transmission data. Fig. 17A is one example of a data structure of the first normal-time transmission data, whose description is omitted here since it overlaps the description in the first embodiment. Fig. 17B is one example of a data structure of the first abnormal-time transmission data 011. The first abnormal-time transmission data 011 may adopt a data structure in which a living body digital signal and a first processed data are continuously recorded for each sensor, such as arranging, for example, a living body digital signal 511 of a first sensor, a first processed data 611 of the first sensor, a living body digital signal 711 of a second sensor, and a first processed data 811 of the second sensor after a header 111, an abnormality signal 211, a wearer identification number 311, and a device identification number 411. The data structure shown in Fig. 17A is merely one example. Hence, the order of the data shown in Fig. 17A may be changed. As for the data structure as well, any data structure such as a hierarchical type, a network type, or a relational type structure may be adopted. Thus, the data structure is not limited to the form shown in Figs. 13A to 13C.

In Fig. 3, first of all, the sensor control unit 66 carries out a control such that, of the sensor groups 5-1 to 5-N included in the sensor unit 12, power is constantly supplied to the sensor determined as the first living body information detecting sensor to be driven in accordance with a request made by the external living body information monitoring device 3 or host server 4, and the sensor determined as the second living body information detecting sensor which is either in a power-save state or controlled so as to be intermittently driven is controlled in accordance with a preset operation control method. Alternatively, when the first living body information detecting sensor and the second living body information detecting sensor are each intermittently driven, the respective living body information detecting sensors are controlled in accordance with a preset operation control method.

At the time when the living body information monitoring device 3 has received the first abnormal-time transmission data, or at the time when the host sensor 4 has received a second abnormal-time transmission data that will be described later, the sensor control unit 66 of the living body information detecting terminal 2 controls the sensor operation as follows in response to a request made either by a living body information detecting terminal control unit 53 that will be described later or by a living body information control unit 54 that will be described later.

The sensor control unit 66 carries out a preset control for, from among the second living body information detecting sensors, the sensor determined so as to implement an operation control method different from that performed during normal conditions, in accordance with a request made by the external living body information monitoring device 3 or host server 4. As for an example of the preset control, for example, when the second living body information detecting sensor is in a power-save state during normal conditions, that sensor is controlled so as to be supplied with power. When the second living body information detecting sensor is intermittently driven during normal conditions, the measuring terminal, the measuring time, and the sampling frequency are controlled. Since definitions of the terms "measuring terminal", "measuring time", and "sampling frequency" described herein are the same as those shown in the first embodiment, a description thereof is omitted here in order to avoid a repetitive description. In particular, the measuring interval is controlled so as to be shorter than during normal conditions, the measuring time is controlled so as to be longer than that during normal conditions, and the data sampling frequency is controlled so as to be higher than that during normal conditions.

Moreover, when the sensor control unit 66 performs a control such that the second living body information detecting sensor is controlled according to a control method different from that used during normal conditions, the first judging unit 86 may also compare an output of the second living body information detecting sensor with the preset value range recorded in the ROM 46-1 to judge whether or not that output indicates abnormality.

In addition, a feature of the above-mentioned example resides in that the sensor control unit 66 controls the sensor operation in accordance with a request made by the external living body information monitoring device 3 or host server 4. However, the living body information detecting terminal 3 itself may also independently control the operations of the sensors using the sensor control unit 66 in response to the judgment results from the first judging unit 86. Since a method of controlling the operation at this time is the same as the above-mentioned method, its description is omitted here in order to avoid a repetitive description.

In addition, in the above-mentioned example, the description has been given with respect to the control method for the sensor control unit 66 which is employed when the data detected by the first living body information detecting sensor is outside the range of the preset value and hence judged to be abnormal. However, when the data detected either by the first living body information detecting sensor or by the second living body information detecting sensor falls within the range of the preset value again, the sensor control unit 66 can return the operation of the first living body information detecting sensor or the second living body information detecting sensor back to a preset reference operation control state.

Also, the above-mentioned living body information detecting terminal 2 includes an event button (not shown). Although, in the above-mentioned example, the operation of outputting the output signal of the first living body information detecting sensor is used as a trigger with which the method of controlling the operation of the second living body information detecting sensor is changed by the above-mentioned sensor control unit 66, an operation of depressing the even button may alternatively serve as the trigger.

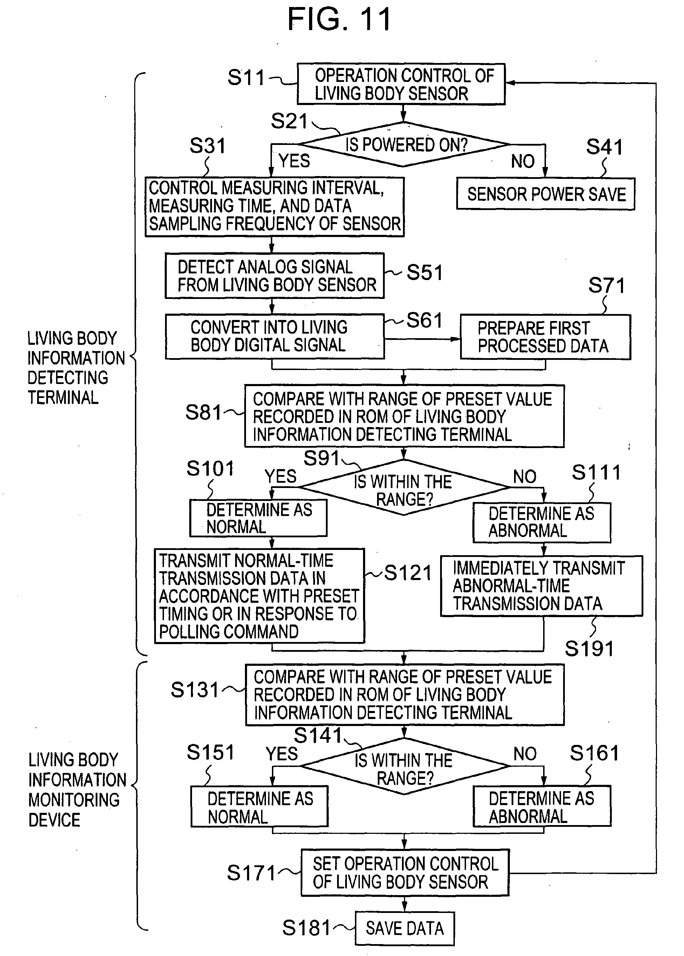

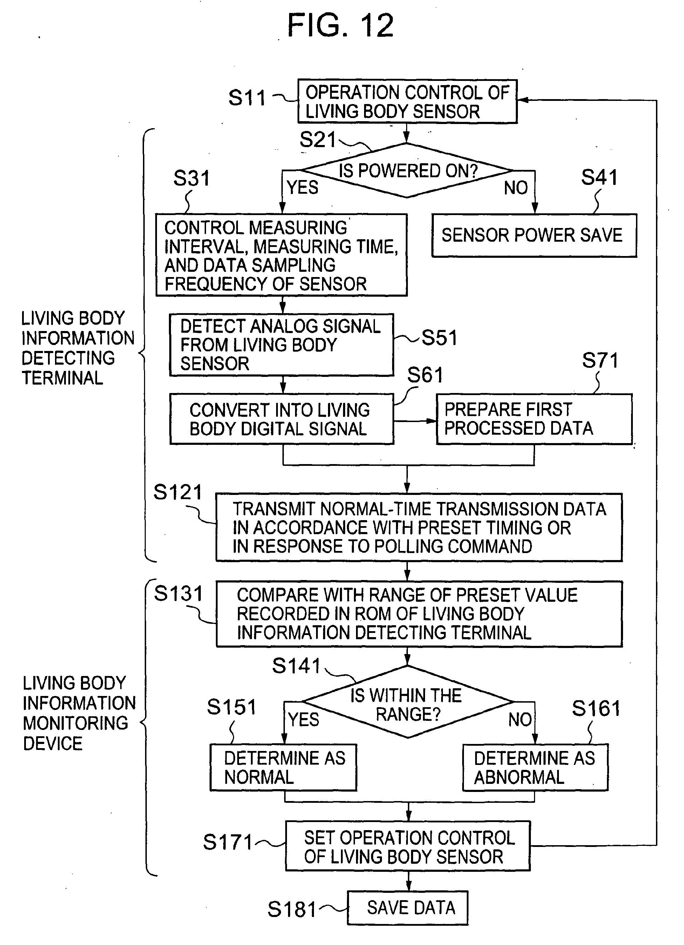

The features of the function and the configuration of the living body information detecting terminal control system 1 of the present invention have been described for each constituent element of the system until now. Next, in Fig. 11 and Fig. 12, the above-mentioned control flow between the above-mentioned living body information detecting terminal 2 and the above-mentioned living body information monitoring device 3 will hereinbelow be described with reference to flow charts.

Fig. 11 is an example of a basic form of the control flow between the living body information detecting terminal 2 and the living body information monitoring device 3 in the living body information detecting terminal control system 1. First of all, in S11, the operations of the living body information detecting sensors previously provided in the living body information detecting terminal 2 are controlled in accordance with a command issued from the living body information monitoring device 3. In S21, it is judged whether or not the power supply should be turned ON. When it is determined in S21 that the power supply should be turned OFF, in S41, no power is supplied to the living body information detecting sensor concerned, thus attaining a sensor power-save state. On the other hand, when it is determined in S21 that the power supply should be turned ON, in S31, the measuring intervals, the measuring time, and the data sampling frequency of the sensor concerned are controlled according to the above-mentioned operation control contents. Next, in S51, an analog signal is detected from the living body information detecting sensor being driven, and is then converted into a living body digital signal in S61 to generate the first processed data in S71. Then, in S81, the first data obtained by combining the living body digital signal and the first processed data which have been obtained in S61 and S71, respectively, is compared with the preset value range recorded in the ROM of the living body information detecting terminal. If it is judged in S91 that the first data falls within the range, then in S101, the first data is judged to be normal. Then, in S121, the normal-time transmission data is transmitted at a preset timing, or in response to a polling command issued from the living body information monitoring device. On the other hand, if it is judged in S91 that the first data is outside the range, then in S111, the first data is judged to be abnormal. Then, in S191, the abnormal-time transmission data is immediately transmitted. Then, the first data which has been transmitted in S121 or in S191 is compared with the preset value range recorded in the ROM of the monitoring terminal in S131. If it is judged in S141 that the first data falls within the range, then in S151, the first data is judged to be normal. On the other hand, if it is judged in S141 that the first data is outside the range, then in S161, the first data is judged to be abnormal. Then, in S171, the operation control contents of the sensors are set according to the above-mentioned operation control contents. Then, in S11, the operations of the living body information detecting sensors included in the living body information detecting terminal are controlled. In addition, in S181, the living body information monitoring device can temporarily store the data therein.

Fig. 12 is one modified example of the control flow between the living body information detecting terminal 2 and the living body information monitoring device 3 in the living body information detecting terminal control system 1. Fig. 12 shows a control flow in which no judgment is carried out in the living body information detecting terminal, data detected and processed in the living body information detecting terminal is transmitted in the form of the first transmission data to the living body information monitoring device, and judgments are collectively carried out in the living body information monitoring device. Thus, this control flow is a control flow in which steps S81, S91, S101, S111, and S191 of Fig. 11 are eliminated. Since the processing contents of S11 to S181 of Fig. 12 are the same as those of respective steps of Fig. 11, these steps are designated with the same reference symbols as those used in Fig. 11, and a description thereof is omitted here in order to avoid a repetitive description.

One example of the basic form and one modified example of the control flow have been shown in the form of flow charts of Fig. 11 and Fig. 12, respectively. However, the control flow is not intended to be limited to the above-mentioned control flows, and hence a control flow may be structured so as to reflect modifications of the above-mentioned structures.

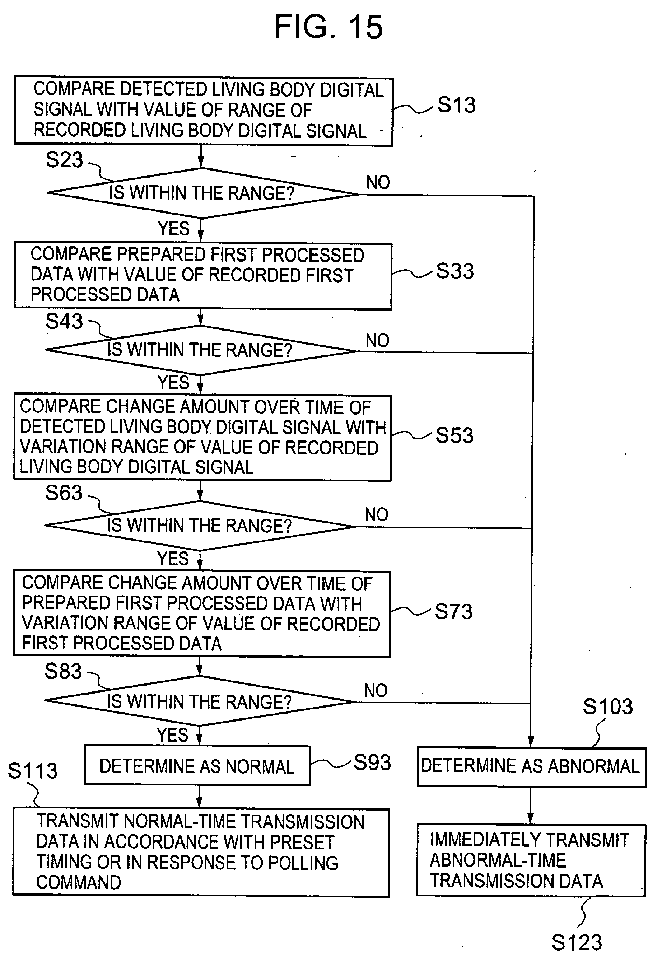

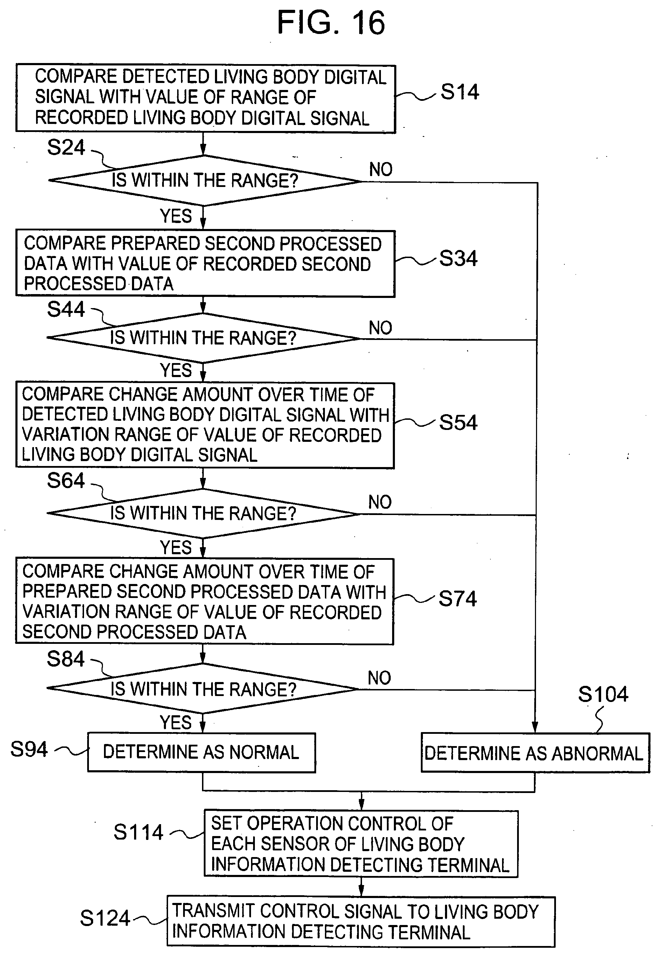

In addition, in Fig. 15 and Fig. 16, judgment flows in the first judging unit included in the living body information detecting terminal 2, and the second judging unit included in the living body information monitoring device 3 are shown in the form of flow charts, respectively.

The judgment flow in the first judging unit included in the living body information detecting terminal 2 will now be described with reference to Fig. 15. First of all, in S13, the detected living body digital signal is compared with the value range of the living body digital signal which is recorded in the ROM. If it is judged in S23 that the detected living body digital signal is outside the range, then in S103, the detected living body digital signal is judged to be abnormal. Then, in S123, the abnormal-time transmission data is immediately transmitted. On the other hand, if it is judged in S23 that the detected living body digital signal falls within the range, then in S33, the first processed data is compared with the value range of the first processed data which is recorded in the ROM. If it is judged in S43 that the first processed data is outside the range, then in S103, the first processed data is judged to be abnormal. Then, in S123, the abnormal-time transmission data is immediately transmitted. On the other hand, if it is judged in S43 that the first processed data falls within the range, then in S53, a change amount over time of the detected living body digital signal is compared with a variation range of the value of the living body digital signal which is recorded in the ROM. If it is judged in S63 that the change amount over time is outside the variation range, then in S103, the change amount over time is judged to be abnormal. Then, in S123, the abnormal-time transmission data is immediately transmitted. On the other hand, if it is judged in S63 that the change amount over time falls within the variation range, then a change amount over time of the first processed data is compared with a variation range of the value of the first processed data which is recorded in the ROM. If it is judged in S83 that the change amount over time is outside the variation range, then in S103, the change amount over time is judged to be abnormal. Then, in S123, the abnormal-time transmission data is immediately transmitted. On the other hand, if it is judged in S83 that the change amount over time falls within the variation range, then in S93, the change amount over time is judged to be normal. Then, in S113, the normal-time transmission data is transmitted at a preset timing, or in response to a polling command issued from the outside.

The judgment flow in the second judging unit included in the living body information monitoring device 3 will now be described with reference to Fig. 16. Since the judgment flow shown in Fig. 16 is the same as that shown in Fig. 15 except that the judgment is carried out for the second processed data in S34 and S74, the operation control for the sensors included in the living body information detecting terminal is set in S114, and a control signal is transmitted to the living body information detecting terminal in S124, its description is omitted here in order to avoid a repetitive description.

One example of the basic form and one modified example of the judgment flow have been shown in the form of flow charts in Fig. 15 and Fig. 16, respectively. However, the judgment flow is not intended to be limited to each of the above-mentioned judgment flows, and hence a judgment flow may be structured so as to reflect modifications of the above-mentioned structures.

Moreover, of the sensor groups 5-1 to 5-N constituting the sensor unit 12, the control for verifying a breakdown condition is carried out for especially the second living body information detecting sensor in accordance with a request made by the external living body information monitoring device or server. The judgment of the breakdown condition is carried out in the above-mentioned first judging unit 86. A method for the judgment will be described later.

In a case where the control for verifying a breakdown condition is carried out for a sensor in the sensor control unit 66, the first judging unit 86 judges whether an output signal of from the sensor, which is obtained when power is supplied to the sensor concerned, is normal or abnormal to thereby judge a breakdown condition of the sensor concerned. With respect to a judgment criterion for a breakdown condition of a sensor, for example, a normal initial signal obtained when power is supplied to each sensor is recorded in the ROM 46-1, and a signal obtained by converting into a digital signal a sensor output signal obtained when power is actually supplied to the sensor concerned, is then compared with the normal initial signal recorded in the ROM 46-1. Then, if the digital signal and the normal initial signal coincide with each other or the digital signal has an error of equal to or smaller than 10 % with respect to the normal initial signal, then the digital signal can be judged to be normal, while if the digital signal has a dispersion of equal to or larger than 10 % or no digital signal can not be obtained, then the digital signal can be judged to be in a failure state. The above-mentioned range of the dispersion for detection of a failure is merely one example, and hence such a range is not limited to the above-mentioned range.

Data on the value range preset as a normal range of the first data that contains therein at least any one of the above-mentioned living body digital signal or the above-mentioned first processed data with respect to each of the above-mentioned living body information detecting sensors is recorded in the ROM 46-1 of the first memory unit 46 in addition to identification information specific to a wearer.

Figs. 13A to 13C show data 09 previously recorded in the ROM 46-1 of the living body information detecting terminal 2, and one example of its data structure. Roughly speaking, the data 09 contains therein at least any one of a header 19, a personal information data cell group 29, a judgment criteria data cell group 39, and a device information data cell group 49. Of those, one example of the contents of the personal information data cell group 29 is shown in Fig. 13A. Personal information of a wearer such as a wearer identification number, a name, date of birth, an address, a telephone number, a blood group, and anamnesis of a wearer, and a name, a contact address and the like of family doctor may be recorded in the personal information data cell group 29. Likewise, one example of the contents of the criterion data cell group 39 is shown in Fig. 13B. References serving as the criteria for judgment, such as an operational expression of data processing as will be described later, a value range of the living body digital signal, a value range of the first processed data, a variation range of a value of the living body digital signal, and a variation range of a value of the first processed data may be recorded in the criterion data cell group 39. Moreover, one example of the contents of the device information data cell group 49 is shown in Fig. 13C. For example, an identification number of a device, an identification number of a living body information monitoring device as a transmission destination of data, data on the timing with which the first data temporarily recorded in the RAM 46-2 is transmitted from the first transmitting/receiving unit 76 to the external living body information monitoring device or server, a list of sensors connected to the living body information detecting terminal 2, and the like may be recorded in the device information data cell group 49. Figs. 13A to 13C show merely one example of each of the data contents and the data structure. Thus, the data contents enumerated in Figs. 13A to 13C may be changed, and any data structure such as a hierarchical type, a network type, or a relational type structure may be adopted. Thus, the data structure or the like is not limited to the form shown in Figs. 13A to 13C.

Since a normal range of the first data is set similarly to a value range preset as a normal range of the second data, its description is omitted here in order to avoid a repetitive description.

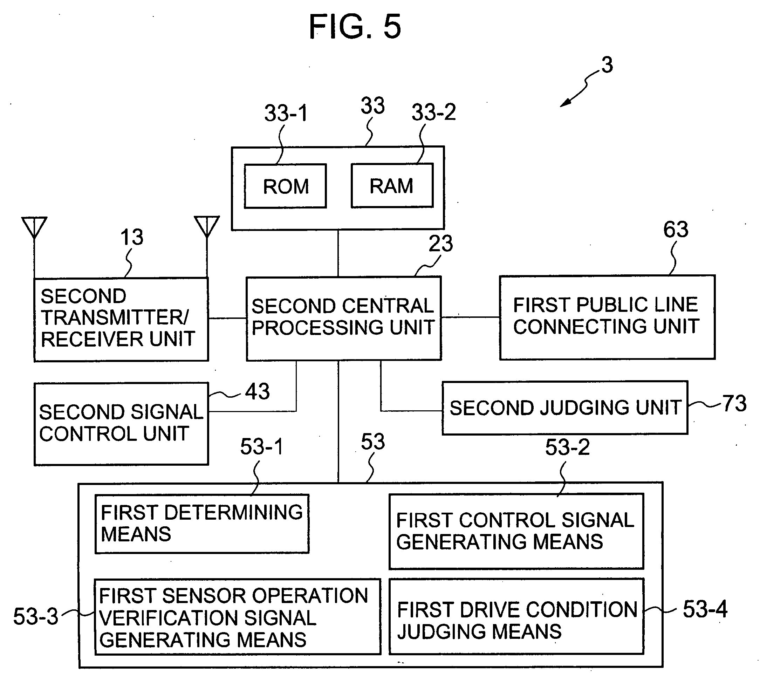

Fig. 5 is a block diagram showing a configuration of the above-mentioned living body information monitoring device 3 shown in Fig. 1, and illustrates a modified example of the living body information monitoring device in the living body information detecting terminal control system of the present invention.

As can be seen by comparing Fig. 5 with Fig. 4, the configuration shown in Fig. 5 is different from that shown in Fig. 4 only in the configuration of the first living body information detecting terminal control unit. Otherwise, the configuration of Fig. 5 is completely identical to that of Fig. 4. Thus, the constituent elements having the same configuration, functions and operations are designated by the same reference numerals, and a description of the functions and the operations is omitted here in order to avoid a repetitive description. Here, a description will be given with respect to the living body information detecting terminal control unit 53 which is different in configuration from that of the living body information monitoring unit 3 shown in Fig. 4, and the second signal control unit 43, the second judging unit 73, and the second central processing unit 23 which operate in manners different from those of the living body information monitoring device 3 shown in Fig. 4.

In the living body information detecting terminal control unit 53 of Fig. 5, the first determining means 53-1 selects and determines between a first living body information detecting sensor which is normally necessary for a wearer of the living body information detecting terminal and a second living body information detecting sensor which is not normally necessary for the wearer, on the basis of identification information specific to the wearer which is received from the living body information detecting terminal. Moreover, at the time when the second transmitting/receiving unit 13 has received an abnormality signal from the living body information detecting terminal 2, the first determining means 53-1 selects and determines a sensor for which an operation control method is to be changed, from among the second living body information detecting sensors. In addition, the first control signal generating means 53-2 generates a control signal for transmitting the control method for the second living body information detecting sensor, which control method is determined by the first determining means, to the living body information detecting terminal 2 in the form of a signal. With respect to the contents of the control, when the selected second living body information detecting sensor is in a power-save state during normal conditions, the control is carried out so as to supply power to the selected second living body information detecting sensor, and when the selected second living body information detecting sensor is intermittently driven during normal conditions, the control is carried out so that the measuring interval is made shorter, the measuring time is made longer, and the data sampling frequency is increased. Moreover, in the above-mentioned example, the abnormality signal from the living body information detecting terminal 2 serves as a trigger with which the above-mentioned first control signal generating means 53-2 is operated. However, an operation of depressing an event button (not shown) included in the living body information monitoring device may alternatively serve as the trigger.

Furthermore, in the living body information detecting terminal control unit 53, a first sensor operation verification signal generating means 53-3 generates a command in accordance with which a breakdown condition is verified for a predetermined time period and at a predetermined timing, such as once a day for 10 seconds, for a second living body information detecting sensor, that is, for the second living body information detecting sensor which is selected and determined as a sensor to be driven at the time when the living body information monitoring device has received an abnormality signal from the second transmitting/receiving unit 13, from among the living body information detecting sensor groups belonging to the sensor unit of the above-mentioned living body information detecting terminal, and transmits the command to the living body information detecting terminal. In addition, a first drive condition judging means 53-4 can judge an operation condition and a breakdown condition of the above-mentioned sensor included in the living body information detecting terminal on the basis of a signal received from the above-mentioned second transmitting/receiving unit 13 in accordance with the breakdown condition verification command.

In addition, the second judging unit 73 and the second central processing unit 23 can compare a first data, which is obtained from a first living body information detecting sensor of the living body information detecting sensor groups included in the living body information detecting terminal, with a preset value range recorded in the ROM 33-1 for the first data from the second living body information detecting sensor to judge true abnormality. The preset value range is the same as that preset as the normal range shown in the first embodiment, and a judgment criterion in the second judging unit 73 is also the same as that in the second judging unit 73 described in the first embodiment. Hence, their descriptions are omitted here in order to avoid repetitive descriptions.

In addition, at the time when the first data has been judged as the true abnormality by the second judging unit 73 and the second central processing unit 23, the second signal control unit 43 can immediately transmit the second transmission data to the host server 4 through the first public line connecting unit 63. In addition to the second transmission data which is transmitted from the living body information monitoring unit 3 to the host server 4 in case of the true abnormality, a communication signal distinguished according to a level of abnormality may also be transmitted. On the other hand, when the first data has not been judged as the true abnormality by the second judging unit 73 and the second processing unit 23, the second data is recorded as normal data in the RAM 33-2, and the second transmission data may be transmitted at a polling timing which is previously recorded in the ROM 33-1 or in accordance with a transmission request made by the host server 4, or the living body information detecting terminal 2 may be requested to measure data again to transmit the resultant first transmission data again.

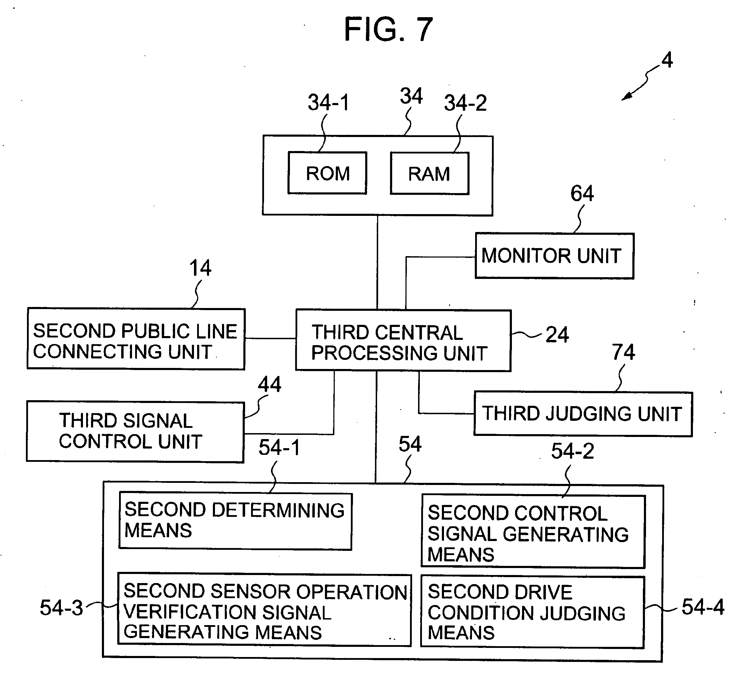

Fig. 7 is a block diagram showing a configuration of the above-mentioned host server 4 of Fig. 1, and illustrates a modified example of the host server in the living body information detecting terminal control system of the present invention.

As can be seen by comparing Fig. 7 with Fig. 6, the configuration shown in Fig. 7 is different from that shown in Fig. 6 only in the configuration of a second living body information detecting terminal control unit. Otherwise, the configuration is completely the same as that of Fig. 6. The living body information detecting terminal control unit 54 of Fig. 7 includes the second determining unit 54-1, the second control signal generating means 54-2, second sensor operation verification signal generating means 54-3, and second drive condition judging means 54-4. Since the operations of those constituent elements are the same as those of the first living body information detecting terminal control unit included in the living body information monitoring device shown in Fig. 6, a description of the operations and the functions is omitted here in order to avoid a repetitive description.

In addition, information on a health state of a wearer of the living body information detecting terminal which has been analyzed by the host server 4 may be periodically sent to the wearer himself/herself. In this case, a method of sending information is not limited as long as this method utilizes communication means such as a telephone, a facsimile, an e-mail, an Internet-mail, or a telegram.

### Third Embodiment

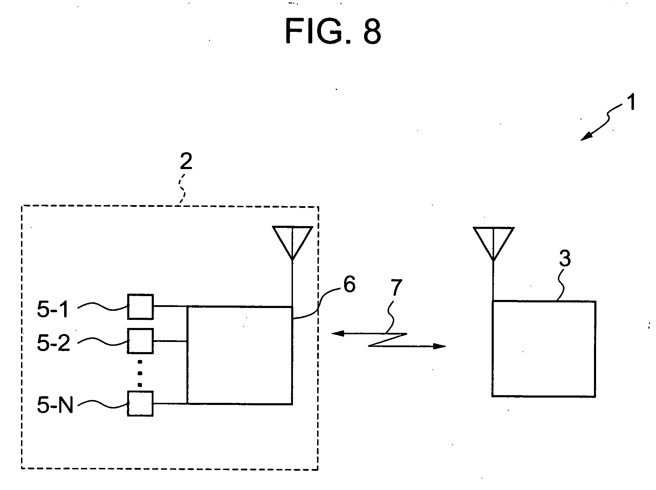

Hereinbelow, a third embodiment of the present invention is described in detail with reference to the drawings. Fig. 8 shows a modified example of the overall construction of the living body information detecting terminal control system according to the present invention. Shown in Fig. 8 is a construction in which the living body information detecting terminal 2 and the living body information monitoring device 3 are connected with each other. As shown in Fig. 8, the living body information monitoring device 3 may not be connected to an external server through a public line. Also, the living body information monitoring device 3 may be connected to the living body information detecting terminal 2 through the public line. Further, the living body information monitoring device 3 may be of the type carried on the wearer or of the stationary type. The constructions, functions, and operations of the respective constituent elements of the living body information detecting terminal 2 and the living body information monitoring device 3 are the same as those of the first embodiment or the second embodiment, and hence a description thereof is omitted here to avoid repetitive description.

### Fourth Embodiment

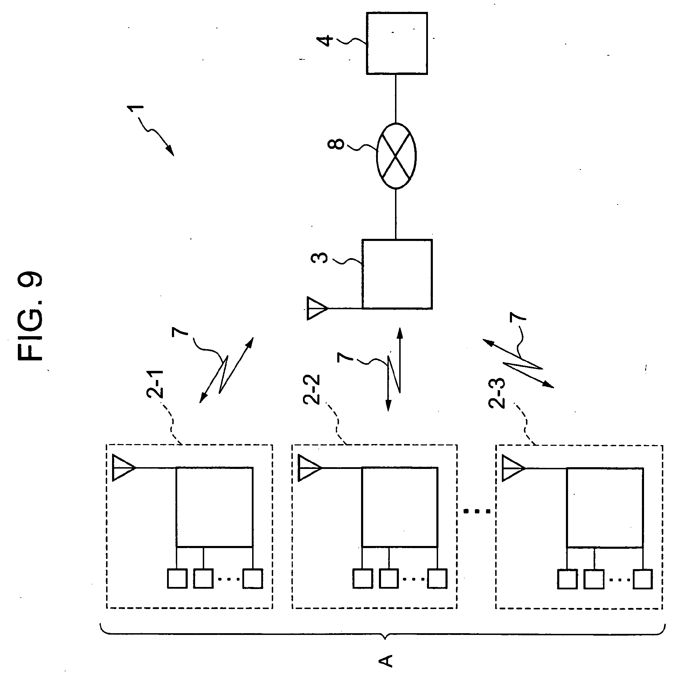

Hereinbelow, a fourth embodiment of the present invention is described in detail with reference to the drawings. Fig. 9 is a modified example of the overall construction of the living body information detecting terminal control system according to the present invention.

As is apparent from the comparison between Fig. 9 and Fig. 1, the overall construction shown in Fig. 9 and the overall construction shown in Fig. 1 are different from each other only in that multiple living body information detecting terminals 2 are provided in the former whereas one living body information detecting terminal 2 is provided in the latter. Otherwise, the two constructions are exactly the same. Accordingly, the constituent elements having the same construction, function, and operation are denoted by the same symbols, and a description thereof is omitted to avoid repetitive description.

Referring to Fig. 9, the living body information detecting terminals 2 are arranged on the wearer in a distributed manner, and the first transmission data from the respective living body information detecting terminals are collectively processed and then temporarily stored. As for the above processing, the same processing as that described in the first embodiment or the second embodiment is performed with respect to each output of each living body information detecting sensor provided to each of the living body information detecting terminals. Thus, a detailed description of the processing is omitted here to avoid repetitive description.

Further, living body information detecting terminal groups 2-1 and 2-2 each consisting of N living body information detecting terminals that are worn on the same wearer (where N is at least 2 or larger), may each consist of at least one battery-driven, portable living body information detecting terminal and at least one stationary living body information detecting terminal that is connected to a commercial power source. Accordingly, when there is no body movement, for example while the wearer is asleep, a sensor connected to a commercial power source, such as a stationary heartbeat meter or respirometer, is used as the sensor that is driven at all times, and the battery-driven, portable living body information detecting terminal is maintained in a power-save state during normal conditions, whereby the portable living body information detecting terminal can be driven while saving power consumption.

## Claims

1. A living body information detecting terminal control system, comprising:
a living body information detecting terminal comprising:
at least one living body information detecting sensor, the living body information detecting sensor serving to detect a living body information signal of a wearer; and
means for transmitting living body information data to a living body information monitoring device;
the living body information monitoring device that, after receiving the living body information data transmitted from the living body information detecting terminal, stores the living body information data in a storage device within the living body information monitoring device for a predetermined time, and includes means for transmitting the living body information data to a host server through a public line; and
the host server that, after receiving the living body information data transmitted from the living body information monitoring device, stores the living body information data in a storage device within the host server, or displaying the living body information data on a display device connected to the host server directly or through a network,
wherein the living body information detecting terminal control system controls an operation of the living body information detecting terminal itself after the living body information detecting terminal detects the living body information signal.

2. A living body information detecting terminal control system, comprising:
a living body information detecting terminal comprising:
at least one living body information detecting sensor, the living body information detecting sensor serving to detect a living body information signal of a wearer; and
means for transmitting living body information data to a living body information monitoring device;
the living body information monitoring device that, after receiving the living body information data transmitted from the living body information detecting terminal, stores the living body information data in a storage device within the living body information monitoring device for a predetermined time, and includes means for transmitting the living body information data to a host server through a public line; and
the host server that, after receiving the living body information data transmitted from the living body information monitoring device, stores the living body information data in a storage device within the host server, or displaying the living body information data on a display device connected to the host server directly or through a network,
wherein, after receiving the living body information data from the living body information detecting terminal, the living body information monitoring device transmits living body information detecting terminal control data, which is used to control drive of the living body information detecting sensor of the living body information detecting terminal, to the living body information detecting terminal.

3. A living body information detecting terminal control system according to claim 2, wherein the living body information detecting terminal further comprises:
at least one living body information detecting sensor;
an A/D converter unit for converting a living body analog signal obtained from the living body information detecting sensor into a living body digital signal;
a living body digital signal control unit for processing the living body digital signal to prepare living body digital signal processed data, and performing control of the living body digital signal;
a ROM having a data structure comprising at least one of wearer identification information specific to the wearer, a living body information operational expression, and living body information detecting terminal identification information;
a first memory unit comprising a RAM for storing living body digital data composed of the living body digital signal or the living body digital signal processed data for a predetermined time;
a living body information sensor control unit for controlling drive of each living body information detecting sensor composing a living body information detecting sensor unit, based on the living body information detecting terminal control data received from the living body information monitoring device or a control content set with respect to the living body information detecting terminal by the wearer;
a living body information data transmitter/receiver unit for transmitting or receiving data to or from the living body information monitoring device; and
a first central processing unit for controlling the A/D converter unit, the first memory unit, the living body digital signal control unit, the living body information sensor control unit, and the living body information data transmitter/receiver unit.

4. A living body information detecting terminal control system according to claim 3, wherein:
the living body information detecting terminal further comprises:
a first living body information data judging unit for comparing the living body digital data with a preset value range and outputting a comparison result; and
a living body information detecting sensor control unit for controlling the drive of the living body information detecting sensor based on the comparison result; and
the living body information detecting sensor control unit controls each living body information detecting sensor based on living body information detecting terminal control data received from the living body information monitoring device, a judgment value obtained from the first living body information data judging unit, or the control content set by the wearer.

5. A living body information detecting terminal control system according to claim 4, wherein the living body digital signal control unit of the living body information detecting terminal comprises:
a living body digital data processing function for performing data processing on the living body digital signal; and
an normal-time living body information transmitting function for storing the living body digital signal processed data obtained by the living body digital data processing function or living body digital data composed of the living body digital signal in the RAM for a predetermined time, and at a predetermined timing or when receiving a polling command transmitted by the living body information monitoring device, transmitting a first normal-time transmission data composed of at least one of the living body digital data and the wearer identification information to the living body information monitoring device.

6. A living body information detecting terminal control system according to claim 5, wherein the living body information detecting terminal further comprises an abnormal-time living body information transmitting function for:
in a case where a value of the living body digital data is judged to be outside the preset value range in the first living body information data judging unit, outputting an abnormality signal indicating an abnormality from the first living body information data judging unit of the living body information detecting terminal, and immediately transmitting a first abnormal-time transmission data composed of the abnormality signal, the wearer identification information, and the living body digital data; and
in a case where the value of the living body digital data is judged to be within the preset value range in the first living body information data judging unit, deleting the abnormal-time transmission data, or storing the abnormal-time transmission data in the RAM for a predetermined time, and at a predetermined timing or when receiving the polling command transmitted by the living body information monitoring device, transmitting the first normal-time transmission data to the living body information monitoring device.

7. A living body information detecting terminal control system according to claim 2, wherein the living body information detecting terminal further comprises:
at least one normal-time driving living body information detecting sensor that is driven during normal conditions; and
at least one power-save living body information detecting sensor that is laid in a power-save state with no power supply during normal conditions.

8. A living body information detecting terminal control system according to claim 2, wherein the living body information detecting terminal further comprises:
at least one normal-time driving living body information detecting sensor that is driven during normal conditions; and
at least one power-save living body information detecting sensor that is intermittently driven during normal conditions.

9. A living body information detecting terminal control system according to claim 7, wherein, in a case where an output value of the first living body information data judging unit is an abnormal output value, the living body information sensor control unit supplies power to the power-save living body information detecting sensor and drives the power-save living body information detecting sensor.

10. A living body information detecting terminal control system according to claim 8, wherein, in a case where an output value of the first living body information data judging unit is an abnormal output value, the living body information sensor control unit performs control by a control method based on at least one of a measuring interval, a measuring time, and a data sampling frequency for an intermittent driving living body information detecting sensor.

11. A living body information detecting terminal control system according to claim 2, wherein the living body information detecting terminal selects a first living body information detecting sensor from the living body information sensors based on an output result from the first living body information data judging unit, and transmits the selection result to the living body information monitoring device through the living body information data transmitter/receiver unit.

12. A living body information detecting terminal control system according to claim 2, wherein the living body information detecting terminal further comprises a call button that enables a sensor operation control by being pressed.

13. A living body information detecting terminal control system according to claim 2, wherein the living body information detecting terminal transmits the living body information data to the living body information monitoring device based on the living body information signal detected by the living body information detecting sensor, or controls the drive of the living body information detecting sensor based on an instruction from the living body information detecting terminal itself.

14. A living body information detecting terminal control system according to claim 2, wherein at least one living body information detecting sensor of sensors composing a living body information detecting sensor unit of the living body information detecting terminal is a sensor for detecting a pulse.

15. A living body information detecting terminal control system according to claim 2, wherein at least one living body information detecting sensor of sensors composing a living body information detecting sensor unit of the living body information detecting terminal is a sensor for detecting a blood sugar level or a blood glucose concentration.

16. A living body information detecting terminal control system according to claim 2, wherein at least one living body information detecting sensor of sensors composing a living body information detecting sensor unit of the living body information detecting terminal is a sensor for detecting a moving state of the wearer.

17. A living body information detecting terminal control system according to claim 2, wherein the living body information monitoring device comprises:
a second transmitter/receiver unit for transmitting/receiving the living body information data to/from the living body information detecting terminal;
a living body digital signal processed data preparing unit for processing the living body digital data received from each living body information detecting sensor to prepare a second living body digital signal processed data;
a second memory unit comprising:
a ROM having data with a data structure composed of at least one of:
living body information monitoring device identification information specific to the living body information monitoring device;
an operational expression for data processing;
a value range preset for the living body information data received from each living body information detecting sensor or second living body information data composed of the second living body information processed data; and
a table for identifying the wearer based on the wearer identification information that is recorded in the ROM of the living body information detecting terminal; and
a RAM for storing the second living body information data for a predetermined time;
a second living body information data judging unit for judging a control method for the living body information monitoring device, and when receiving a first abnormal-time transmission data transmitted by the living body information monitoring device, comparing the second living body information data with the preset value range stored in the ROM for judgment;
a first living body information detecting terminal control unit that prepares living body information detecting terminal control data for controlling the living body information detecting terminal based on a judgment result from the second living body information data judging unit;
a first public line connecting unit for connecting to the host server through a public line; and
a second central processing unit for controlling the second transmitter/receiver unit, the second memory unit, the second judging unit, the second signal control unit, the first living body information detecting terminal control unit, and the first public line connecting unit.

18. A living body information detecting terminal control system according to claim 17, wherein:
when the second living body information data is judged as being outside the value range preset by the second living body information data judging unit, the living body information monitoring device transmits a signal indicating an abnormality and a second abnormal-time transmission data composed of the wearer identification information, the living body information detecting terminal identification information, and the second living body information data, to the host server through the first public line connecting unit; and
when the second living body information data is judged as being within the value range preset by the second living body information data judging unit, the living body information monitoring device stores the second living body information data in the RAM for a predetermined time, and at a predetermined timing or in accordance with reception of a polling command received from the host server, transmits a second living body information data stored in the RAM and a second normal-time transmission data composed of the wearer identification information and the living body information detecting terminal identification information, to the host server through the first public _line connecting unit.

19. A living body information detecting terminal control system according to claim 17, wherein, in the living body information monitoring device in a case where the second living body information data is judged as being outside the value range preset by the second living body information data judging unit, and the second abnormal-time transmission data is transmitted to the host server:
the second living body information data judging unit has a function of determining an abnormal level taking as a criterion at least one of a type of the living body information detecting sensor exhibiting an abnormality and a differential of the second living body information data with respect to the preset value range;
the living body digital signal processed data preparing unit adds an abnormality signal corresponding to the abnormal level to the second abnormal-time transmission data; and
the second transmitter/receiver unit transmits the second abnormal-time transmission data.

20. A living body information detecting terminal control system according to claim 17, wherein:
the first living body information detecting terminal control unit comprises a first sensor determining means for, based on the wearer identification information received from the second transmitter/receiver unit, selecting and determining one of a first normal-time driving living body information detecting sensor and the second living body information detecting sensor that is in a power-save state with no power supplied during normal conditions or is intermittently driven, from among the living body information detecting sensors included in the living body information detecting terminal; and
the first living body information detecting terminal control unit transmits the second abnormal-time transmission data to the living body information detecting terminal based on a determination result from the sensor determining means.

21. A living body information detecting terminal control system according to claim 17, wherein the first living body information detecting terminal control unit further comprises:
a first determining means for, when the second transmitter/receiver unit receives the first abnormal-time transmission data from the living body information detecting terminal, selecting and determining the living body information detecting sensor to be driven from among the second living body information detecting sensors that are included in the living body information detecting terminal and are in a power-save state during normal conditions; and
a first control signal generating means for generating a signal that drives the selected second living body information detecting sensor.

22. A living body information detecting terminal control system according to claim 17, wherein the first living body information detecting terminal control unit further comprises:
a first determining means for, when the second transmitter/receiver unit receives the first abnormal-time transmission data from the living body information detecting terminal, selecting and determining the living body information detecting sensor to be changed in at least one of a measuring interval, a measuring time, and a data sampling frequency, from among the second living body information detecting sensors that are intermittently driven during normal conditions within the living body information detecting terminal; and
a first control signal generating means for, based on a result from the first determining means, generating a signal for changing at least one of the measuring interval, the measuring time, and the data sampling frequency of the selected second living body information detecting sensor.

23. A living body information detecting terminal control system according to claim 17, wherein the living body information monitoring device further comprises a call button that enables operation control of a sensor by being pressed.

24. A living body information detecting terminal control system according to claim 2, wherein the living body information monitoring device performs a sensor operation control in response to a signal detected by the living body information detecting terminal.

25. A living body information detecting terminal control system according to claim 2, wherein, when an output from the first living body information detecting sensor is judged as being outside the preset value range, the living body information sensor control unit within the living body information detecting terminal or the first living body information detecting terminal control unit within the living body information monitoring device performs a control such that a measuring interval of the second living body information detecting sensor becomes shorter than the measuring interval required during normal conditions.

26. A living body information detecting terminal control system according to claim 2, wherein, when an output from the first living body information detecting sensor is judged as being outside the preset value range, the living body information sensor control unit within the living body information detecting terminal or the first living body information detecting terminal control unit within the living body information monitoring device performs a control such that a measuring time of the second living body information detecting sensor becomes longer than the measuring time required during normal conditions.

27. A living body information detecting terminal control system according to claim 2, wherein, when an output from the first living body information detecting sensor is judged as being abnormal, the living body information sensor control unit within the living body information detecting terminal or the first living body information detecting terminal control unit within the living body information monitoring device performs a control such that a data sampling frequency of the second living body information detecting sensor becomes higher than the data sampling frequency required during normal conditions.

28. A living body information detecting terminal control system according to claim 2, wherein the living body information sensor control unit included in the living body information detecting terminal or the first living body information detecting terminal control unit included in the living body information monitoring device causes control of the second living body information detecting sensor to return to a preset reference control state, in a case where an output from the first living body information detecting sensor is judged as being outside the preset value range by the first judging unit or the second judging unit, and the output from the first living body information detecting sensor is again judged as being within the preset value range with respect to the second living body information detecting sensor in which at least one of the measuring interval, the measuring time, and the data sampling frequency is controlled.

29. A living body information detecting terminal control system according to claim 2, wherein the first living body information detecting terminal control unit further comprises:
a first sensor operation verification signal generating means for generating a verification signal that serves to regularly verify a breakdown condition or a drive condition of each living body information detecting sensor within the living body information detecting terminal; and
a first drive condition judging means for, after the living body information detecting sensor control unit included in the living body information detecting terminal verifies a breakdown condition or a drive condition of each living body information detecting sensor in response to the operation verification signal and receives a sensor operation verification result signal to be transmitted, judging the breakdown condition or the drive condition of the sensor from the living body information detecting sensor operation verification result signal.

30. A living body information detecting terminal control system according to claim 2, wherein the host server comprises:
a third memory unit comprising:
a ROM in which the wearer identification information transmitted from the living body information monitoring device, a table for identifying the wearer from the living body information monitoring device identification information, and the polling timing are previously recorded; and
a RAM for storing a second living body information data transmitted from the living body information monitoring device;
a second public line connecting unit for receiving the second normal-time transmission data or the second abnormal-time transmission data that are transmitted from the living body information monitoring device through the public line, and transmitting a polling command to the living body information monitoring device at the polling timing previously recorded in the ROM provided to the host server;
a display unit for displaying the second normal-time transmission data or the abnormal-time transmission data that are received from the living body information monitoring device;
a third signal control unit for performing a signal control by determining whether the second normal-time transmission data or the abnormal-time transmission data that are received from the living body information monitoring device are stored in the memory unit or displayed on the display unit;
a second living body information detecting terminal control unit for generating a signal for controlling the living body information detecting terminal; and
a third central processing unit for controlling the third memory unit, the second public line connecting unit, the display unit, the third signal control unit, and the second living body information detecting terminal control unit.

31. A living body information detecting terminal control system according to claim 30, wherein the second living body information detecting terminal control unit comprises:
the second determining means for, based on the identification information specific to the living body information monitoring device and the wearer identification information that are received by the second public line connecting unit, determining one of the first living body information detecting sensor that is driven during normal conditions and the second living body information detecting sensor that is in a power-save state with no power supply or is intermittently driven during normal conditions, from among the living body information detecting sensors included in the living body information detecting terminal; and
the second control signal generating means for generating a sensor control signal that is transmitted directly to the living body information detecting terminal or transmitted to the living body information monitoring device.

32. A living body information detecting terminal control system according to claim 31, wherein the second living body information detecting terminal control unit further comprises:
a second living body information detecting sensor operation verification signal generating means for generating a verification signal that serves to regularly verify a breakdown condition or a drive condition of each living body information detecting sensor included in the living body information detecting terminal; and
a second drive condition judging means for, after the living body information detecting sensor control unit included in the living body information detecting terminal verifies a breakdown condition or a drive condition of each living body information detecting sensor in response to the operation verification signal and receives a living body information detecting sensor operation verification result signal to be transmitted, judging the breakdown condition or the drive condition of the sensor from the living body information detecting sensor operation verification result signal.

33. A living body information detecting terminal control system according to claim 32, wherein, in a case where an output signal from the first living body information detecting sensor exceeds a preset reference value range, or in a case where a difference with respect to an output signal previously outputted from the living body information detecting sensor exceeds a preset variation range, the first judging unit included in the living body information detecting terminal or the second judging unit included in the living body information monitoring device judges the output signal to be abnormal.
